# EUROPEAN PATENT APPLICATION

(11) **EP 4 201 931 A1**
(43) Date of publication of application: **28.06.2023**
(21) Application number: 21858495.1
(22) Date of filing: 30.07.2021
(51) Int. Cl.: C07D 405/04, C07D 405/14, C07D 409/14, C07D 495/04, H01L 51/00, H01L 51/50

(54) **HETEROCYCLIC COMPOUND AND ORGANIC LIGHT-EMITTING DEVICE COMPRISING SAME**

(30) Priority: 18.08.2020 KR 20200103402
(71) Applicant: LT Materials Co., Ltd., Yongin-si, Gyeonggi-do 17118 (KR)
(72) Inventor: MO, Jun-Tae, Yongin-si, Gyeonggi-do 17118 (KR); KIM, Ji-Young, Yongin-si, Gyeonggi-do 17118 (KR); LEE, Young-Jin, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/009969
(87) International publication number: WO 2022/039408

(57) **Abstract**

The present application provides a heterocyclic compound, an organic light emitting device including the heterocyclic compound in an organic material layer, and a composition for an organic material layer.

## Description

### [Technical Field]

This application claims priority to and the benefits of Korean Patent Application No. 10-2020-0103402, filed with the Korean Intellectual Property Office on August 18, 2020, the entire contents of which are incorporated herein by reference.

The present specification relates to a heterocyclic compound, and an organic light emitting device including the same.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### Prior Art Documents

### Patent Documents

US Patent No. 4,356,429

### [Disclosure]

### [Technical Problem]

The present disclosure is directed to providing a heterocyclic compound, and an organic light emitting device including the same.

### [Technical Solution]

One embodiment of the present application provides a heterocyclic compound represented by the following Chemical Formula 1. 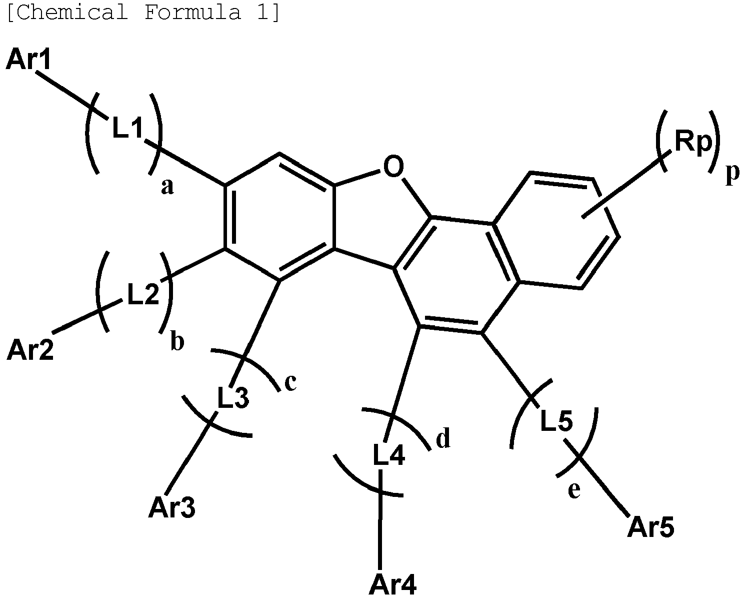 In Chemical Formula 1,
L1 to L5 are the same as or different from each other, and each independently a direct bond; a substituted or unsubstituted arylene group having 6 to 60 carbon atoms; or a substituted or unsubstituted heteroarylene group having 2 to 60 carbon atoms,
a, b, c, d and e are each an integer of 0 to 3, and when a, b, c, d and e are each 2 or greater, substituents in the parentheses are the same as or different from each other,
Ar1 to Ar5 are the same as or different from each other, and each independently a substituted or unsubstituted aryl group having 6 to 60 carbon atoms; or a substituted or unsubstituted heteroaryl group having 2 to 60 carbon atoms,
at least one of Ar1 to Ar5 is a monocyclic or polycyclic heteroaryl group having 2 to 60 carbon atoms substituted or unsubstituted and including one or more Ns; or an aryl group having 6 to 60 carbon atoms substituted with one or more cyano groups, and
Rp is hydrogen; deuterium; a halogen group; or a substituted or unsubstituted alkyl group having 6 to 60 carbon atoms, p is an integer of 0 to 4, and when p is 2 or greater, substituents in the parentheses are the same as or different from each other.

In addition, another embodiment of the present application provides an organic light emitting device including a first electrode, a second electrode, and one or more organic material layers provided between the first electrode and the second electrode, wherein one or more layers of the organic material layers include the heterocyclic compound represented by Chemical Formula 1.

### [Advantageous Effects]

A heterocyclic compound according to one embodiment of the present application can be used as a material of an organic material layer of an organic light emitting device. The heterocyclic compound can be used as a material of a hole injection layer, a hole transfer layer, a light emitting layer, an electron transfer layer, an electron injection layer, a charge generation layer and the like in an organic light emitting device. Particularly, the heterocyclic compound represented by Chemical Formula 1 can be used as a material of a light emitting layer of an organic light emitting device. In addition, using the heterocyclic compound represented by Chemical Formula 1 in an organic light emitting device is capable of lowering a driving voltage of the device, enhancing light efficiency, and enhancing lifetime properties of the device by thermal stability of the compound.

### [Description of Drawings]

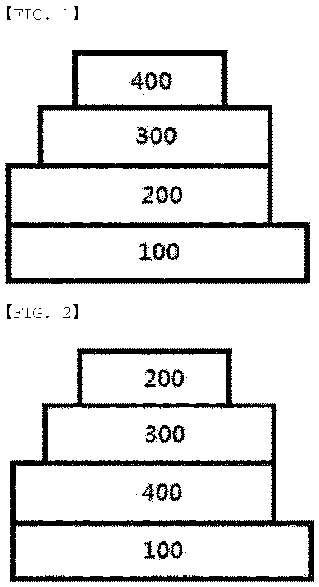

FIG. 1 to FIG. 4 are diagrams each schematically illustrating a lamination structure of an organic light emitting device according to one embodiment of the present application.

### [Mode for Disclosure]

Hereinafter, the present application will be described in detail.

One embodiment of the present application provides a heterocyclic compound represented by the following Chemical Formula 1. 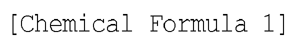 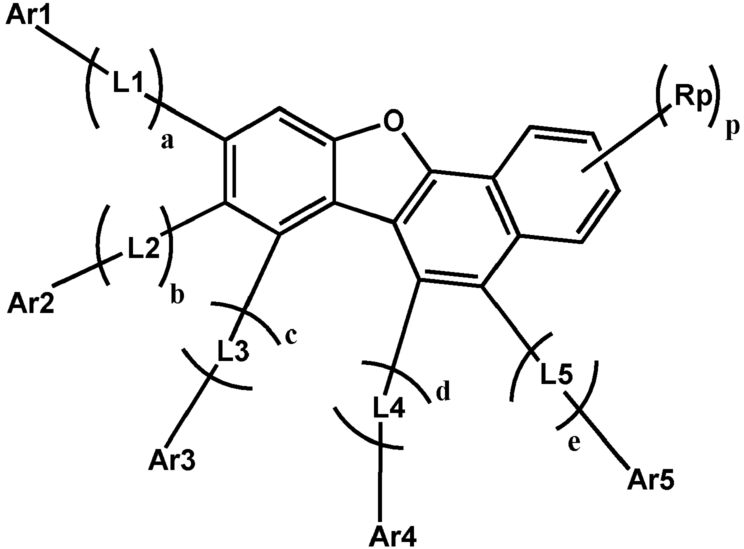 In Chemical Formula 1,
L1 to L5 are the same as or different from each other, and each independently a direct bond; a substituted or unsubstituted arylene group having 6 to 60 carbon atoms; or a substituted or unsubstituted heteroarylene group having 2 to 60 carbon atoms,
a, b, c, d and e are each an integer of 0 to 3, and when a, b, c, d and e are each 2 or greater, substituents in the parentheses are the same as or different from each other,
Ar1 to Ar5 are the same as or different from each other, and each independently a substituted or unsubstituted aryl group having 6 to 60 carbon atoms; or a substituted or unsubstituted heteroaryl group having 2 to 60 carbon atoms,
at least one of Ar1 to Ar5 is a monocyclic or polycyclic heteroaryl group having 2 to 60 carbon atoms substituted or unsubstituted and including one or more Ns; or an aryl group having 6 to 60 carbon atoms substituted with one or more cyano groups, and
Rp is hydrogen; deuterium; a halogen group; or a substituted or unsubstituted alkyl group having 6 to 60 carbon atoms, p is an integer of 0 to 4, and when p is 2 or greater, substituents in the parentheses are the same as or different from each other.

By having a monocyclic or polycyclic heterocyclic group including one or more Ns or an aryl group substituted with a cyano group in the dibenzofuran structure as a substituent, Chemical Formula 1 has an improved electron transfer ability, and, by improving a current flow, an effect of lowering a driving voltage is obtained when using the compound represented by Chemical Formula 1 in a device.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, the "substituted or unsubstituted" means being substituted with one or more substituents selected from the group consisting of deuterium; a cyano group; a halogen group; linear or branched alkyl having 1 to 60 carbon atoms; linear or branched alkenyl having 2 to 60 carbon atoms; linear or branched alkynyl having 2 to 60 carbon atoms; monocyclic or polycyclic cycloalkyl having 3 to 60 carbon atoms; monocyclic or polycyclic heterocycloalkyl having 2 to 60 carbon atoms; monocyclic or polycyclic aryl having 6 to 60 carbon atoms; monocyclic or polycyclic heteroaryl having 2 to 60 carbon atoms; -SiRR'R"; -P(=O)RR'; alkylamine having 1 to 20 carbon atoms; monocyclic or polycyclic arylamine having 6 to 60 carbon atoms; and monocyclic or polycyclic heteroarylamine having 2 to 60 carbon atoms, or being unsubstituted, or being substituted with a substituent linking two or more substituents selected from among the substituents illustrated above, or being unsubstituted, and R, R' and R" are the same as or different from each other and each independently substituted or unsubstituted alkyl having 1 to 60 carbon atoms; substituted or unsubstituted aryl having 6 to 60 carbon atoms; or substituted or unsubstituted heteroaryl having 2 to 60 carbon atoms.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 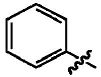 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

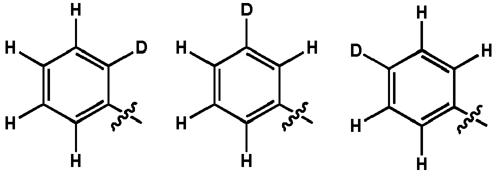

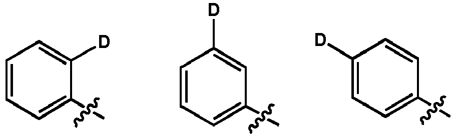

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide group may include a diphenylphosphine oxide group, a dinaphthylphosphine oxide group and the like, but are not limited thereto.

In the present specification, the silyl group is a substituent including Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the spiro group may include any one of groups of the following structural formulae.

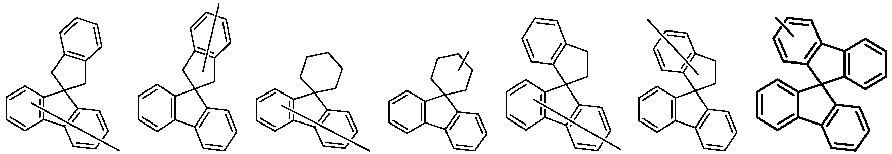

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto. In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except that these are each a divalent group. In addition, the heteroarylene group means the heteroaryl group having two bonding sites, that is, a divalent group. The descriptions on the heteroaryl group provided above may be applied thereto except that these are each a divalent group.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

The heterocyclic compound according to one embodiment of the present application is represented by Chemical Formula 1. More specifically, by having a core structure and structural properties of the substituents as above, the heterocyclic compound represented by Chemical Formula 1 may be used as a material of an organic material layer of an organic light emitting device.

In one embodiment of the present application, L1 to L5 of Chemical Formula 1 are the same as or different from each other, and may be each independently a direct bond; a substituted or unsubstituted arylene group having 6 to 60 carbon atoms; or a substituted or unsubstituted heteroarylene group having 2 to 60 carbon atoms.

In one embodiment of the present application, L1 to L5 are the same as or different from each other, and may be each independently a direct bond; a substituted or unsubstituted arylene group having 6 to 40 carbon atoms; or a substituted or unsubstituted heteroarylene group having 2 to 40 carbon atoms.

In one embodiment of the present application, L1 to L5 are the same as or different from each other, and may be each independently a direct bond; a substituted or unsubstituted arylene group having 6 to 20 carbon atoms; or a substituted or unsubstituted heteroarylene group having 2 to 20 carbon atoms.

In another embodiment, L1 is a direct bond.

In another embodiment, L1 is a phenylene group.

In another embodiment, L1 is a naphthylene group.

In another embodiment, L2 is a direct bond.

In another embodiment, L2 is a phenylene group.

In another embodiment, L2 is a naphthylene group.

In another embodiment, L3 is a direct bond.

In another embodiment, L3 is a phenylene group.

In another embodiment, L3 is a naphthylene group.

In another embodiment, L4 is a direct bond.

In another embodiment, L4 is a phenylene group.

In another embodiment, L4 is a naphthylene group.

In another embodiment, L5 is a direct bond.

In another embodiment, L5 is a phenylene group.

In another embodiment, L5 is a naphthylene group.

In one embodiment of the present application, a, b, c, d and e of Chemical Formula 1 are each an integer of 0 to 3, and when a, b, c, d and e are each 2 or greater, substituents in the parentheses may be the same as or different from each other.

In one embodiment of the present application, Ar1 to Ar5 of Chemical Formula 1 are the same as or different from each other and each independently a substituted or unsubstituted aryl group having 6 to 60 carbon atoms; or a substituted or unsubstituted heteroaryl group having 2 to 60 carbon atoms, and at least one of Ar1 to Ar5 may be a monocyclic or polycyclic heteroaryl group having 2 to 60 carbon atoms substituted or unsubstituted and including one or more Ns; or an aryl group having 6 to 60 carbon atoms substituted with one or more cyano groups.

In one embodiment of the present application, Ar1 to Ar5 are the same as or different from each other and each independently a substituted or unsubstituted aryl group having 6 to 40 carbon atoms; or a substituted or unsubstituted heteroaryl group having 2 to 40 carbon atoms, and at least one of Ar1 to Ar5 may be a monocyclic or polycyclic heteroaryl group having 2 to 40 carbon atoms substituted or unsubstituted and including one or more Ns; or an aryl group having 6 to 40 carbon atoms substituted with one or more cyano groups.

In one embodiment of the present application, Ar1 to Ar5 are the same as or different from each other and each independently a substituted or unsubstituted aryl group having 6 to 20 carbon atoms; or a substituted or unsubstituted heteroaryl group having 2 to 20 carbon atoms, and at least one of Ar1 to Ar5 may be a monocyclic or polycyclic heteroaryl group having 2 to 20 carbon atoms substituted or unsubstituted and including one or more Ns; or an aryl group having 6 to 20 carbon atoms substituted with one or more cyano groups.

In one embodiment of the present application, Ar1 to Ar5 are the same as or different from each other and each independently a substituted or unsubstituted phenyl group; a substituted or unsubstituted biphenyl group; a substituted or unsubstituted naphthyl group; or a monocyclic or polycyclic heteroaryl group having 2 to 60 carbon atoms substituted or unsubstituted and including one or more Ns; or an aryl group having 6 to 60 carbon atoms substituted with one or more cyano groups, and at least one of Ar1 to Ar5 may be a monocyclic or polycyclic heteroaryl group having 2 to 60 carbon atoms substituted or unsubstituted and including one or more Ns; or an aryl group having 6 to 60 carbon atoms substituted with one or more cyano groups.

In one embodiment of the present application, the monocyclic or polycyclic heteroaryl group having 2 to 60 carbon atoms including one or more Ns may be a group represented by the following Chemical Formula 3. 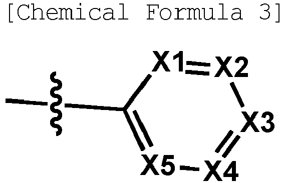 In Chemical Formula 3,
X1 is CR1 or N, X2 is CR2 or N, X3 is CR3 or N, X4 is CR4 or N, X5 is CR5 or N, and at least one of X1 to X5 is N, and
R1 to R5 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -P(=O)R10R12; and NR13R14, or two or more groups adjacent to each other bond to each other to form a substituted or unsubstituted aliphatic or aromatic hydrocarbon ring or heteroring, R10 and R12 to R14 are the same as or different from each other and each independently hydrogen; deuterium; a halogen group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms, and 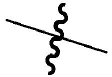 is a site linked to Chemical Formula 1.

In one embodiment of the present application, Chemical Formula 3 may be represented by one of the following Chemical Formulae 3-1 to 3-4. Herein, 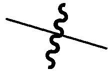 is a site linked to Chemical Formula 1. 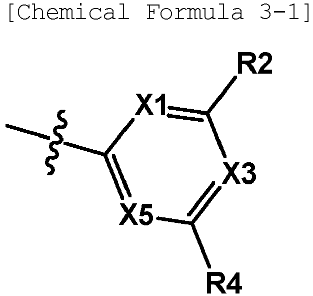 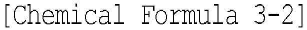 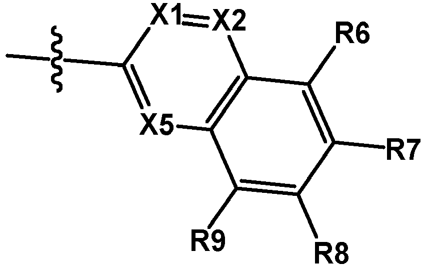 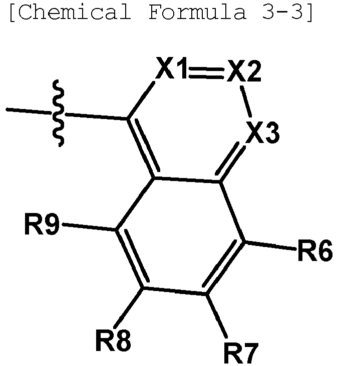 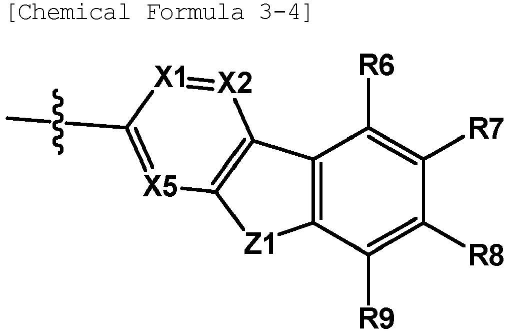
In Chemical Formula 3-1, at least one of X1, X3 and X5 is N, and the rest have the same definitions as in Chemical Formula 3,
in Chemical Formula 3-2, at least one of X1, X2 and X5 is N, and the rest have the same definitions as in Chemical Formula 3,
in Chemical Formula 3-3, at least one of X1 to X3 is N, and the rest have the same definitions as in Chemical Formula Chemical Formula 3,
in Chemical Formula 3-4, at least one of X1, X2 and X5 is N, and the rest have the same definitions as in Chemical Formula 3,
Z1 is O; or S, and
R2, R4 and R6 to R9 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -P(=O)R10R12; and NR13R14, or two or more groups adjacent to each other bond to each other to form a substituted or unsubstituted aliphatic or aromatic hydrocarbon ring or heteroring, and R10 and R12 to R14 are the same as or different from each other and each independently hydrogen; deuterium; a halogen group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms.

In one embodiment of the present application, Chemical Formula 3 may be represented by any one of the following Group 1.

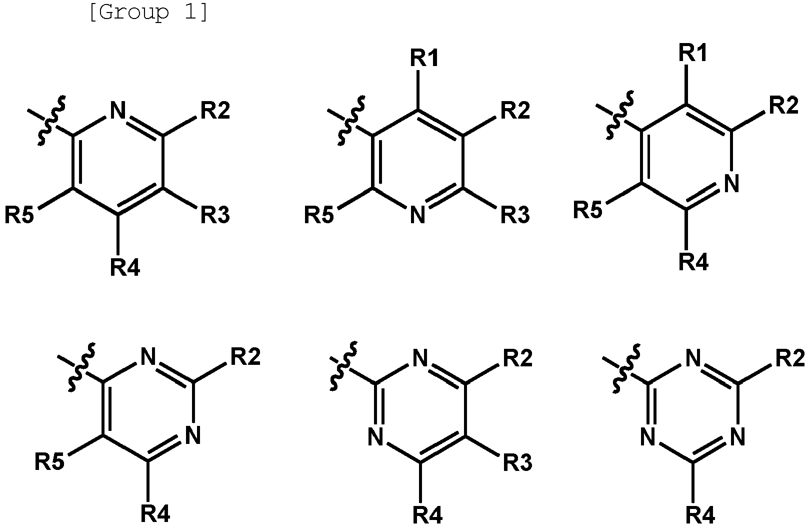

In Group 1, R1 to R5 and 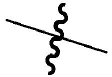 have the same definitions as in Chemical Formula 3.

In another embodiment, Rp of Chemical Formula 1 is hydrogen; deuterium; a halogen group; or a substituted or unsubstituted alkyl group having 6 to 60 carbon atoms, p is an integer of 0 to 4, and when p is 2 or greater, substituents in the parentheses may be the same as or different from each other.

In another embodiment, Rp may be hydrogen; or deuterium.

In another embodiment, Rp is hydrogen.

In one embodiment of the present application, Chemical Formula 1 may be represented by the following Chemical Formula 1-1 or Chemical Formula 1-2. 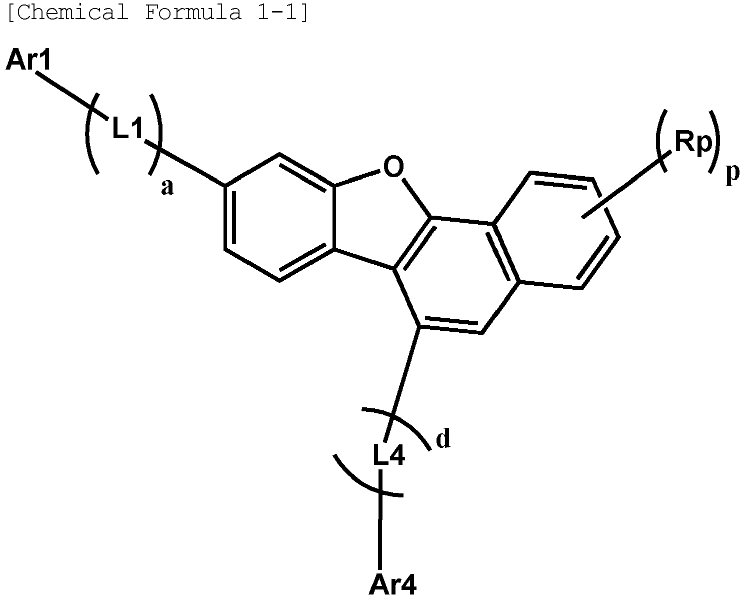 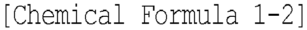 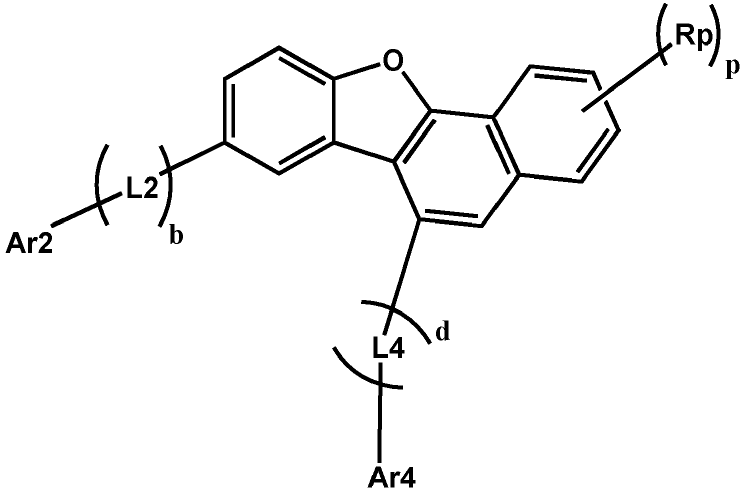 In Chemical Formula 1-1, Ar1 and Ar4 are the same as or different from each other and each independently a substituted or unsubstituted aryl group having 6 to 60 carbon atoms; or a substituted or unsubstituted heteroaryl group having 2 to 60 carbon atoms, at least one of Ar1 and Ar4 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1, and
in Chemical Formula 1-2, Ar2 and Ar4 are the same as or different from each other and each independently a substituted or unsubstituted aryl group having 6 to 60 carbon atoms; or a substituted or unsubstituted heteroaryl group having 2 to 60 carbon atoms, at least one of Ar2 and Ar4 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1.

The heterocyclic compound represented by Chemical Formula 1-1 has a linear and planar shape, and therefore, overlap between the compounds increases accelerating electron transfer. Accordingly, when using the heterocyclic compound represented by Chemical Formula 1-1 in a device, the device may have more superior efficiency.

In addition, the heterocyclic compound represented by Chemical Formula 1-2 has a structure that is very effective in separating HOMO and LUMO electron distribution allowing the compound to have a proper band gap. Accordingly, when using the heterocyclic compound represented by Chemical Formula 1-2 in a device, the device may have a more superior driving effect.

In one embodiment of the present application, Chemical Formula 1 may be represented by any one of the following Chemical Formulae 1-3 to 1-5. 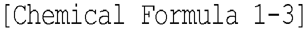 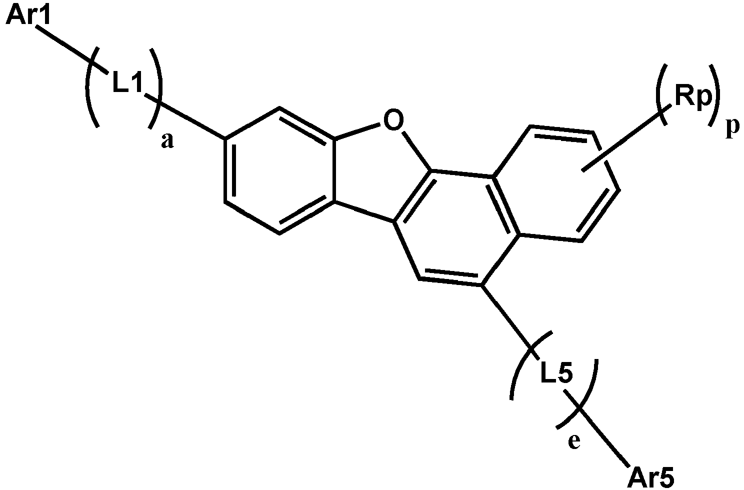 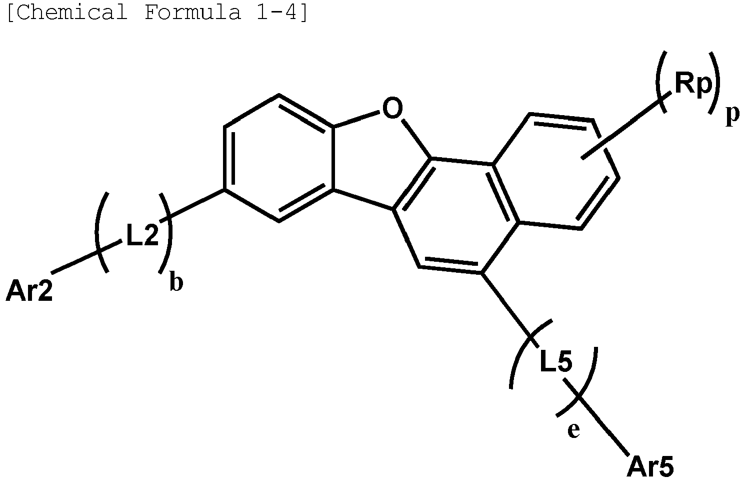 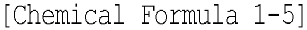 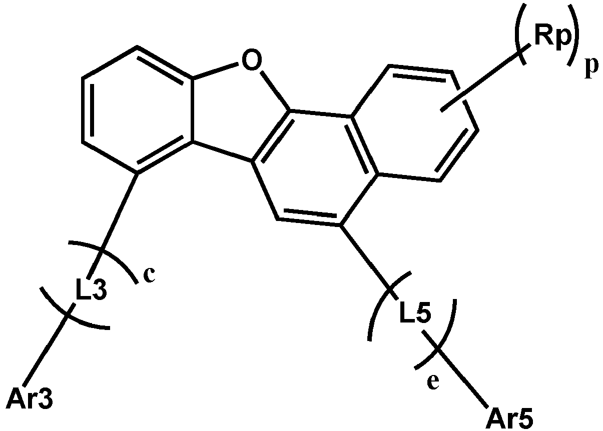
in Chemical Formula 1-3, Ar1 and Ar5 are the same as or different from each other and each independently a substituted or unsubstituted aryl group having 6 to 60 carbon atoms; or a substituted or unsubstituted heteroaryl group having 2 to 60 carbon atoms, at least one of Ar1 and Ar5 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1,
in Chemical Formula 1-4, Ar2 and Ar5 are the same as or different from each other and each independently a substituted or unsubstituted aryl group having 6 to 60 carbon atoms; or a substituted or unsubstituted heteroaryl group having 2 to 60 carbon atoms, at least one of Ar2 and Ar5 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1, and
in Chemical Formula 1-5, Ar3 and Ar5 are the same as or different from each other and each independently a substituted or unsubstituted aryl group having 6 to 60 carbon atoms; or a substituted or unsubstituted heteroaryl group having 2 to 60 carbon atoms, at least one of Ar3 and Ar5 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1.

The heterocyclic compound represented by Chemical Formula 1-3 has a linear and planar shape, and therefore, overlap between the compounds increases accelerating electron transfer. Accordingly, when using the heterocyclic compound represented by Chemical Formula 1-3 in a device, the device may have more superior efficiency.

The heterocyclic compound represented by Chemical Formula 1-4 has a relatively thermally stable structure since the deposition temperature is not high due to less structural interference caused by surrounding functional groups and due to moderate linearity and planarity. Accordingly, when using the heterocyclic compound represented by Chemical Formula 1-4 in a device, the device may have a more superior lifetime.

In addition, the heterocyclic compound represented by Chemical Formula 1-5 has a structure that is very effective in separating HOMO and LUMO electron distribution allowing the compound to have a proper band gap. Accordingly, when using the heterocyclic compound represented by Chemical Formula 1-5 in a device, the device may have a more superior driving effect.

According to one embodiment of the present application, Chemical Formula 1 may be represented by any one of the following compounds, but is not limited thereto.

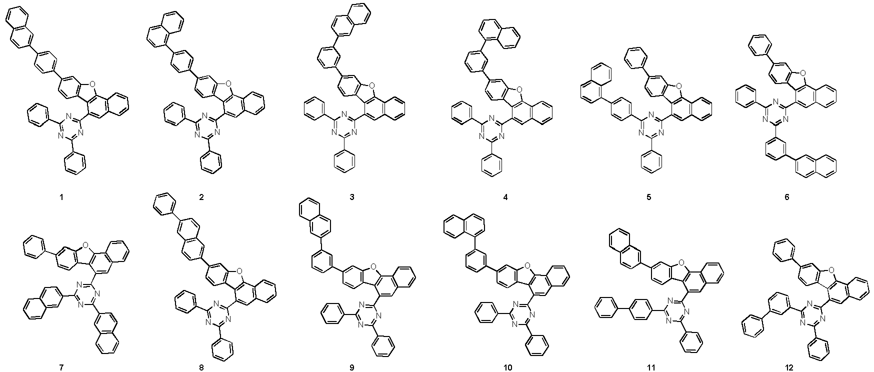

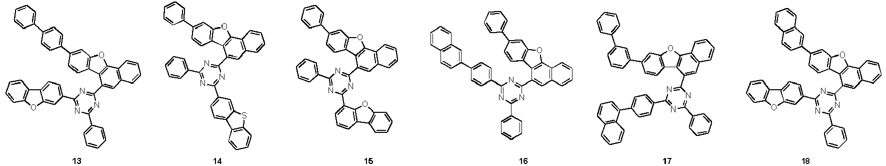

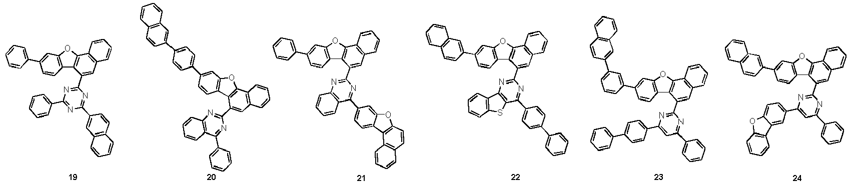

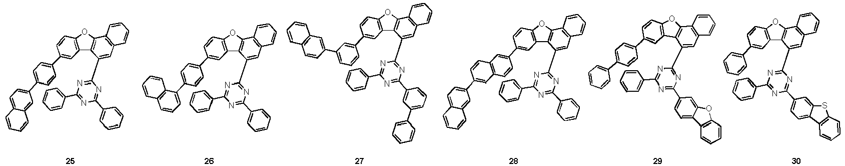

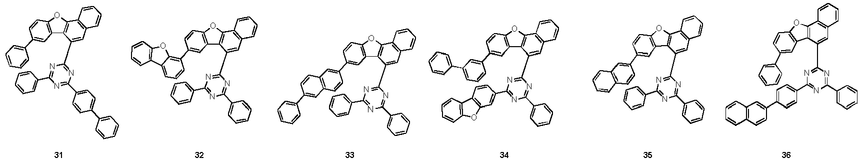

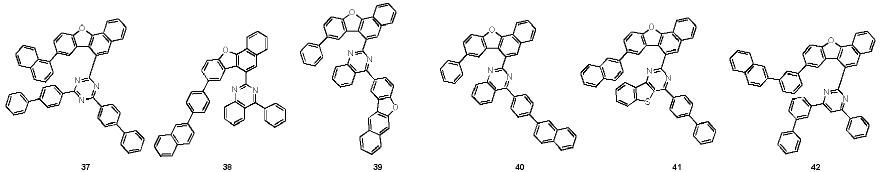

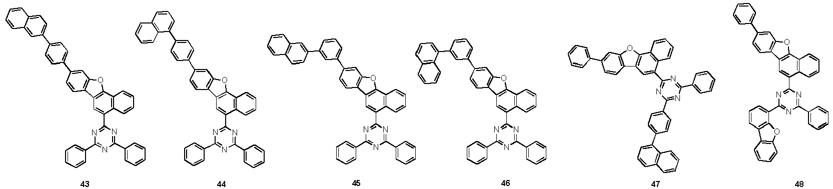

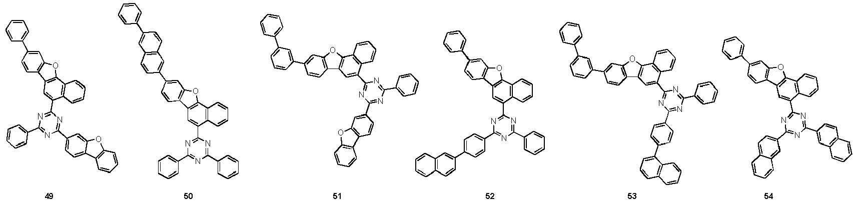

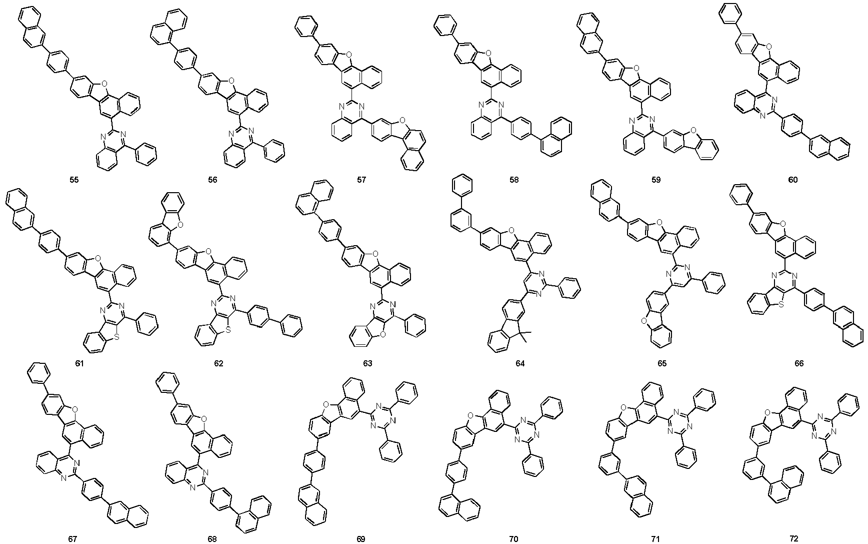

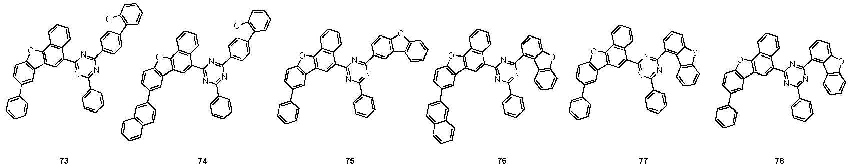

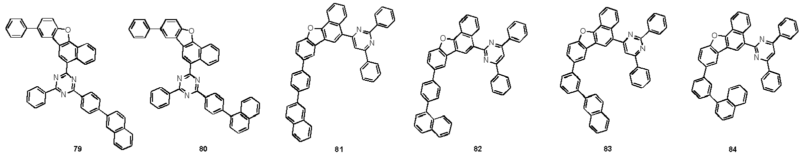

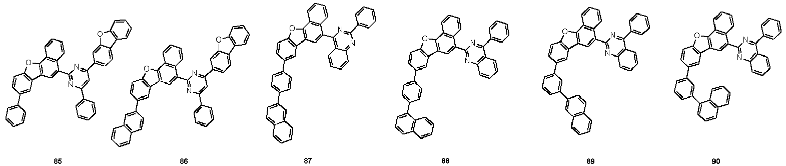

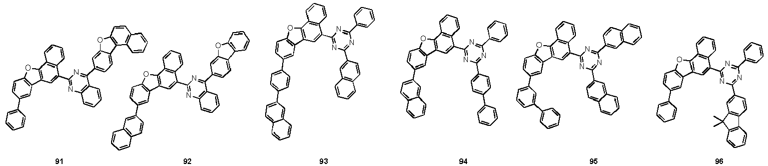

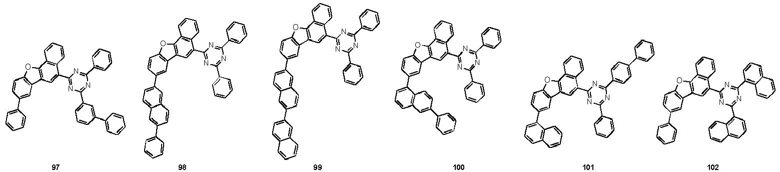

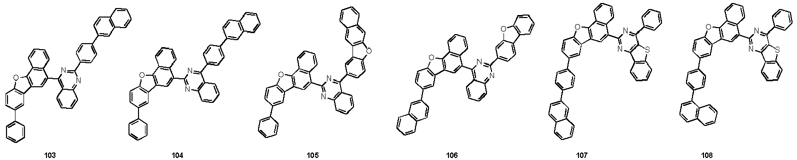

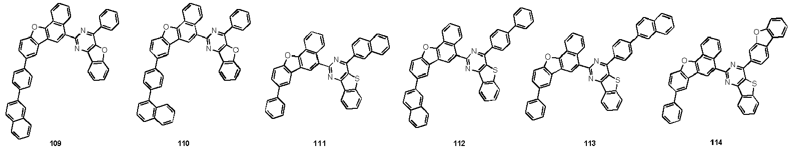

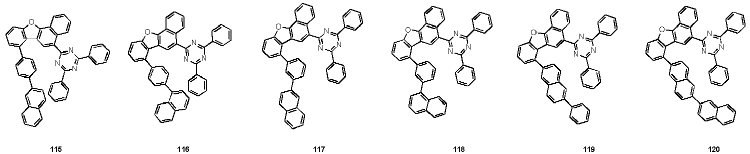

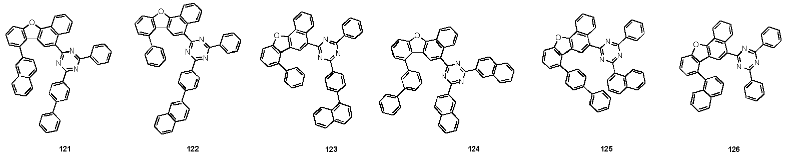

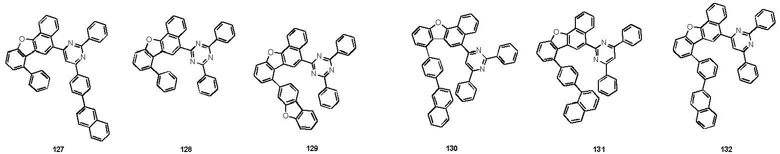

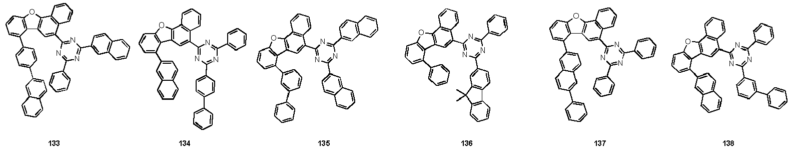

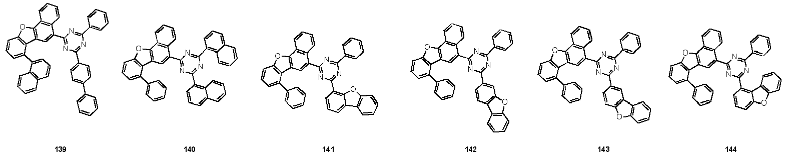

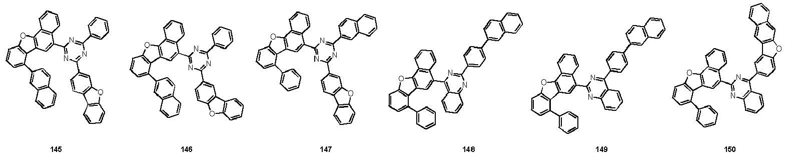

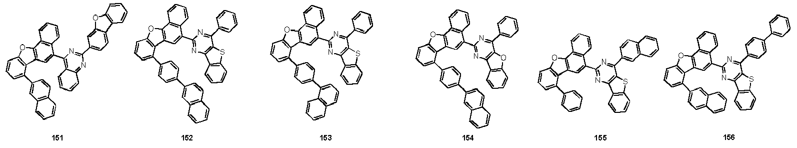

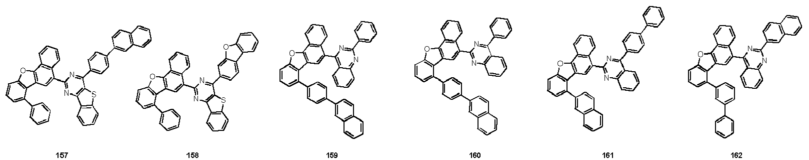

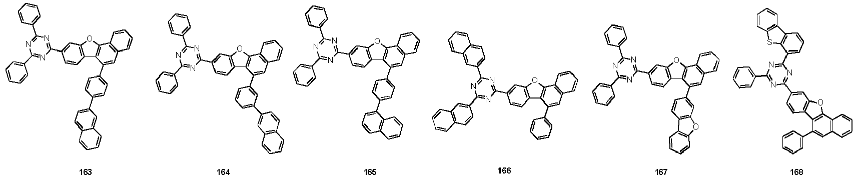

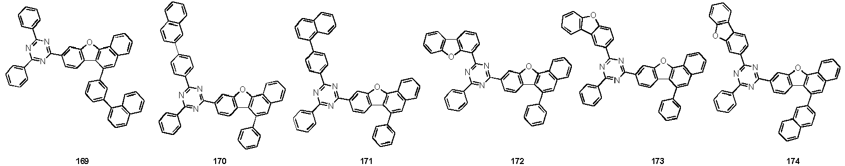

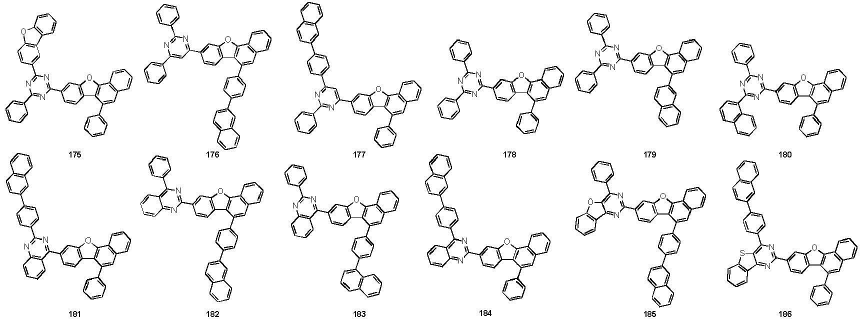

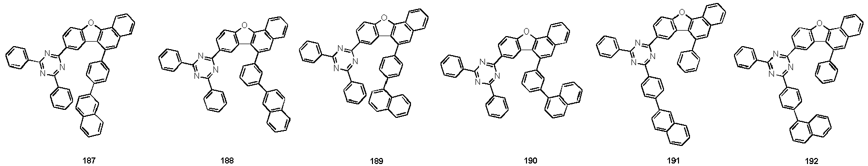

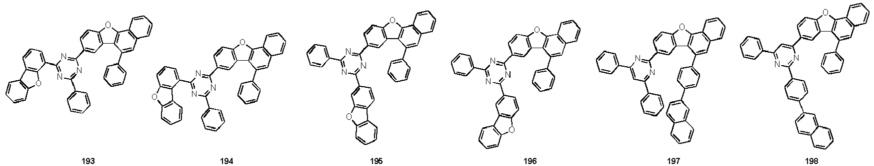

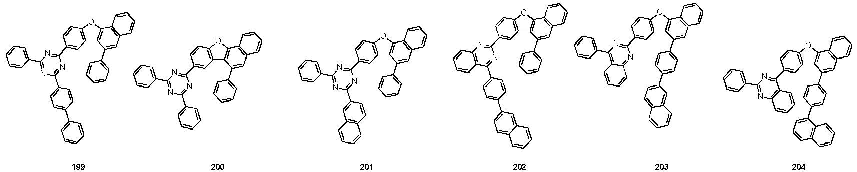

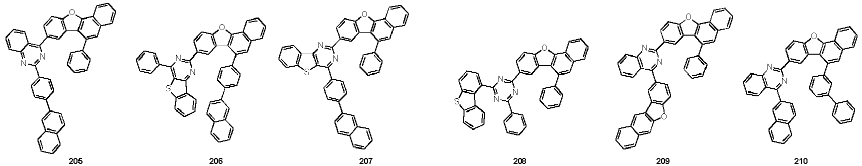

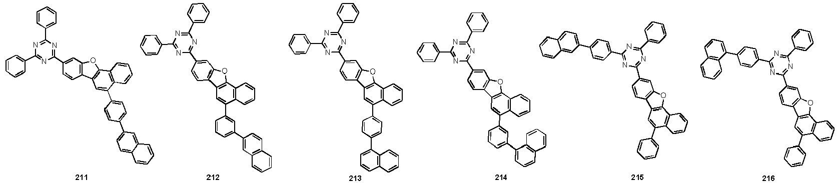

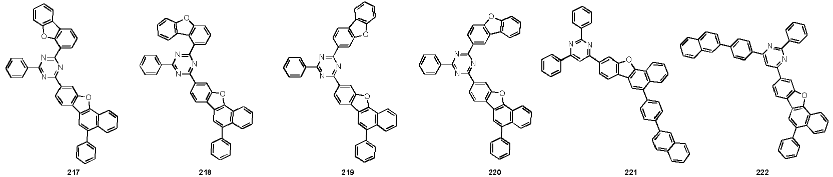

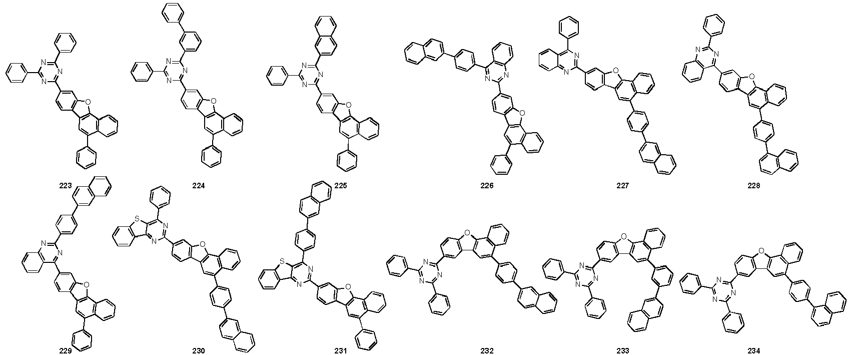

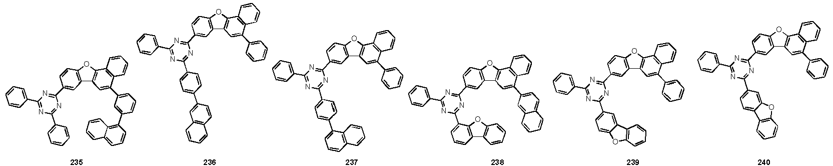

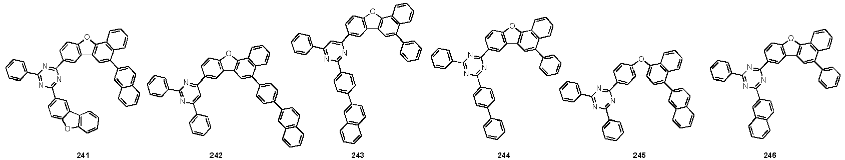

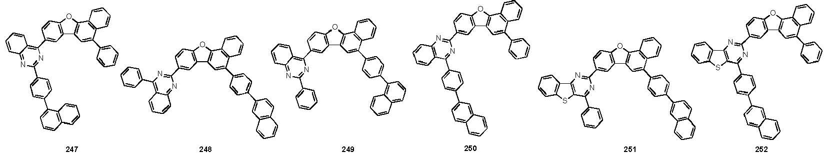

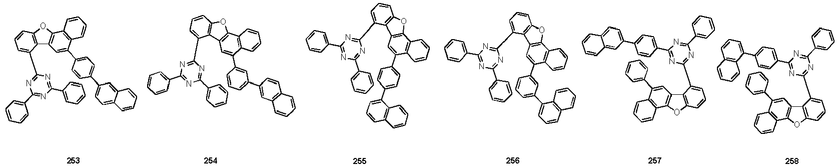

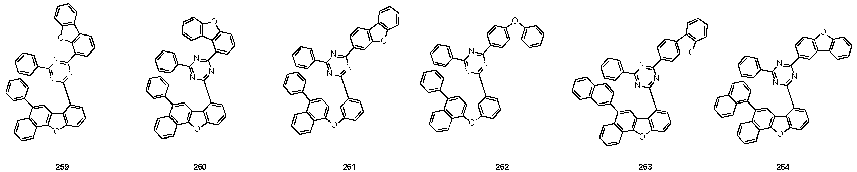

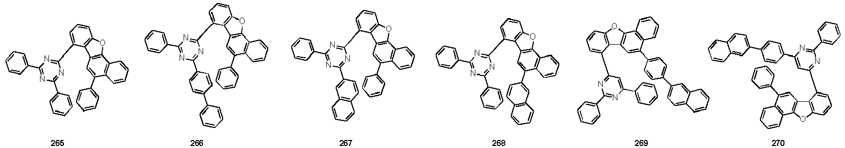

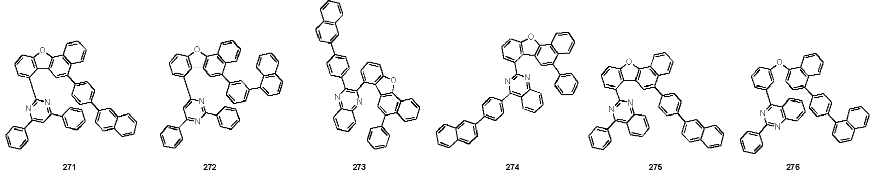

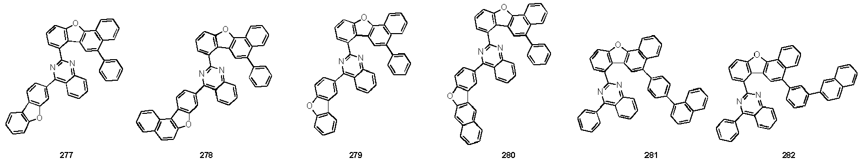

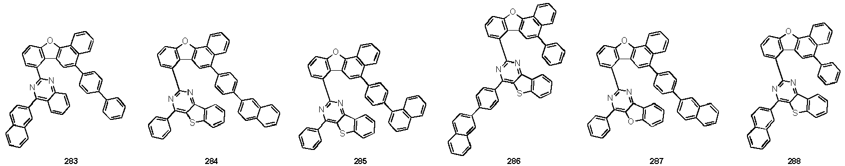

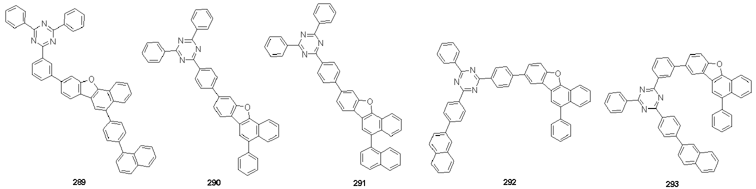

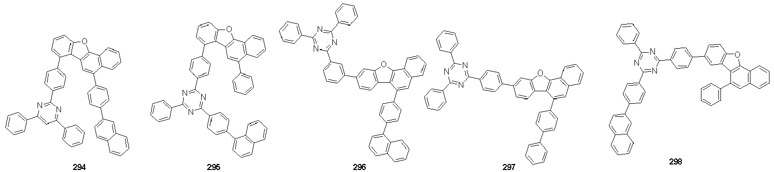

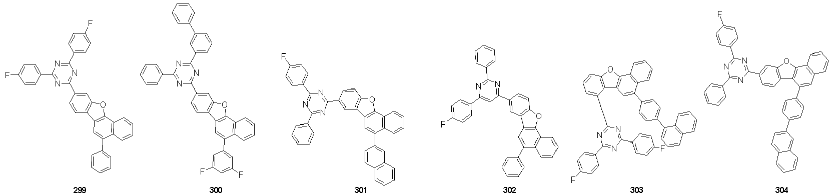

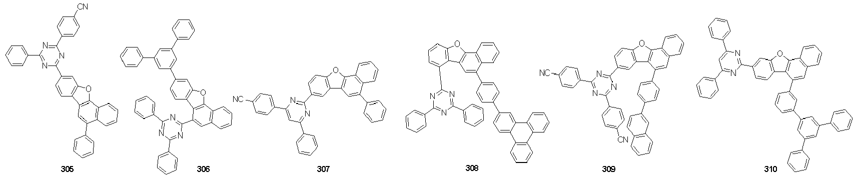

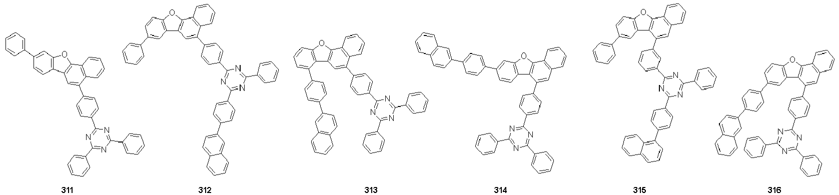

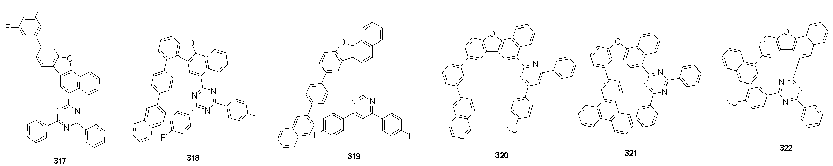

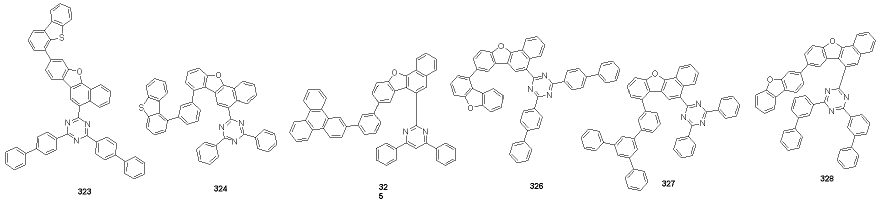

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

Meanwhile, the heterocyclic compound has a high glass transition temperature (Tg), and has excellent thermal stability. Such an increase in the thermal stability becomes an important factor providing driving stability to a device.

The heterocyclic compound according to one embodiment of the present application may be prepared using a multi-step chemical reaction. Some intermediate compounds are prepared first, and from the intermediate compounds, the compound of Chemical Formula 1 may be prepared. More specifically, the heterocyclic compound according to one embodiment of the present application may be prepared based on preparation examples to describe later.

Another embodiment of the present application provides an organic light emitting device including the heterocyclic compound represented by Chemical Formula 1. The "organic light emitting device" may be expressed in terms such as an "organic light emitting diode", an "OLED", an "OLED device" and an "organic electroluminescent device".

The heterocyclic compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

Specifically, the organic light emitting device according to one embodiment of the present application includes a first electrode, a second electrode, and one or more organic material layers provided between the first electrode and the second electrode, and one or more layers of the organic material layers include the heterocyclic compound represented by Chemical Formula 1. When including the heterocyclic compound represented by Chemical Formula 1 in the organic material layer, the organic light emitting device has superior light emission efficiency and lifetime.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a red organic light emitting device, and the heterocyclic compound according to Chemical Formula 1 may be used as a material of the red organic light emitting device.

In one embodiment of the present application, the heterocyclic compound according to Chemical Formula 1 may be used as an N-type host.

In addition, the organic material layer includes one or more light emitting layers, and the light emitting layer includes the heterocyclic compound represented by Chemical Formula 1. When including the heterocyclic compound represented by Chemical Formula 1 in the light emitting layer among the organic material layers, the organic light emitting device has more superior light emission efficiency and lifetime.

In addition, in the organic light emitting device of the present application, the organic material layer may further include any one or more of compounds of the following Group A to Group C.

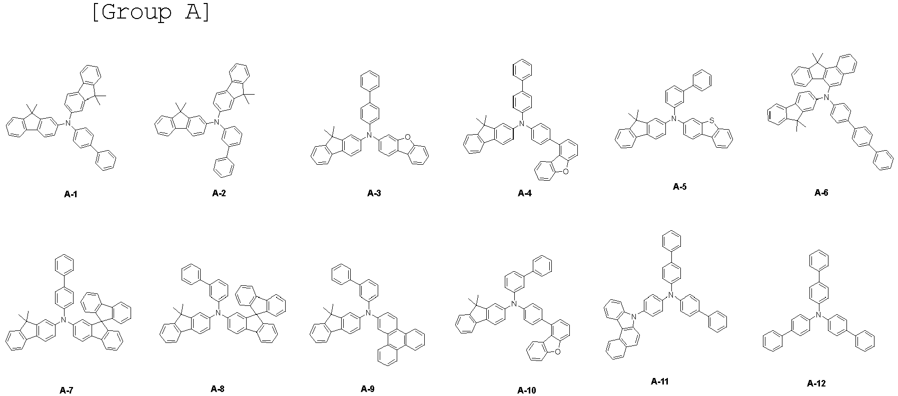

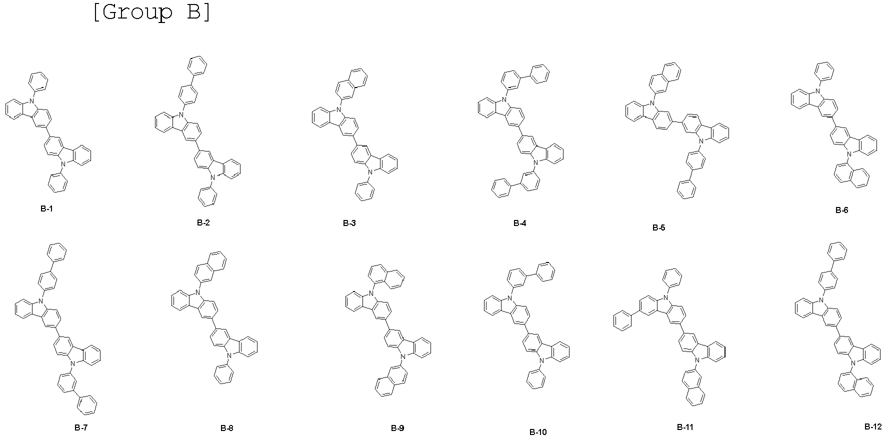

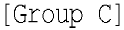

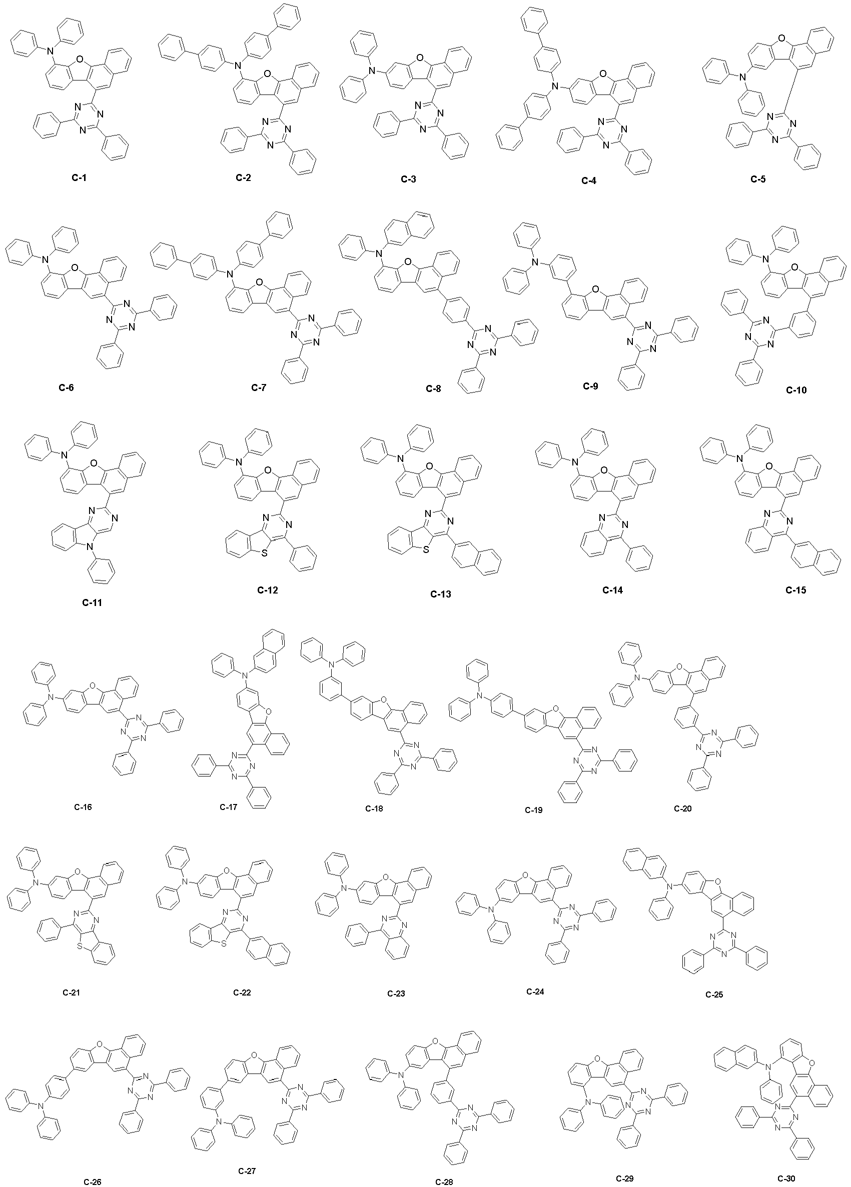

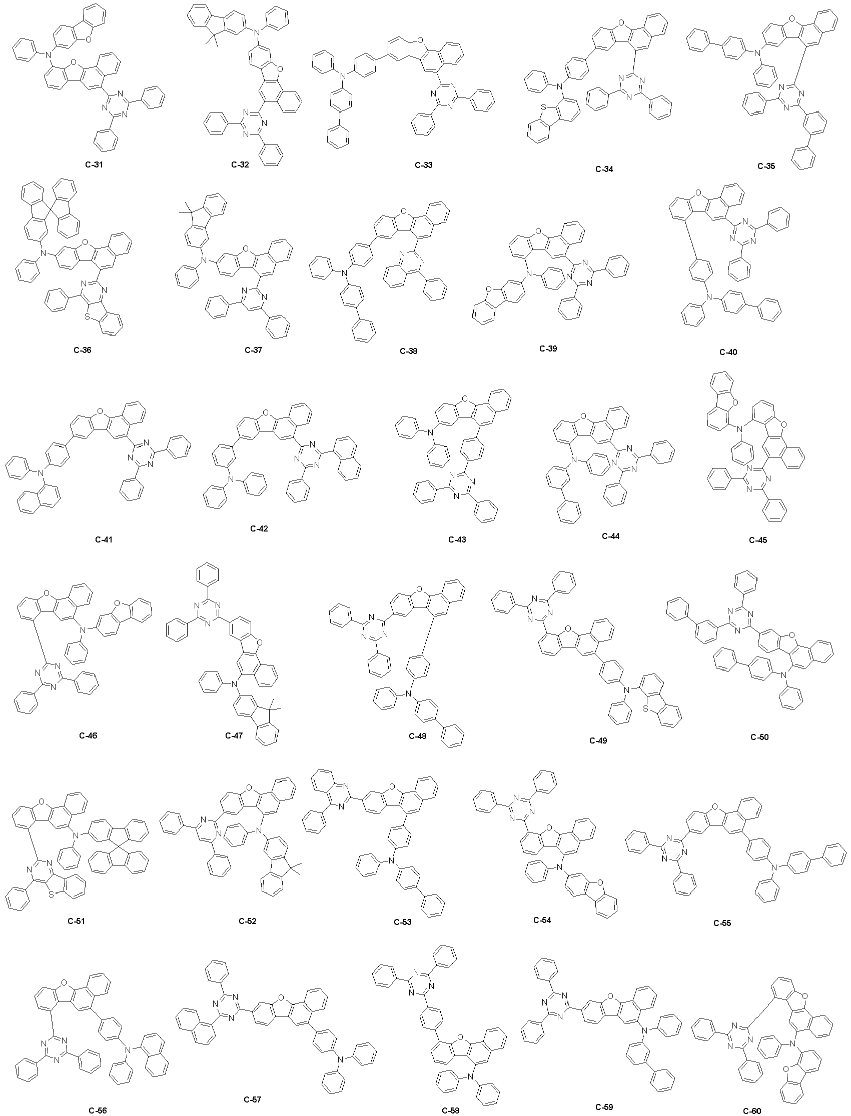

In addition, the organic material layer includes one or more light emitting layers, and the light emitting layer includes the heterocyclic compound represented by Chemical Formula 1 as a first compound, and may further include one of the compounds of Group A to Group C as a second compound. When including the heterocyclic compound represented by Chemical Formula 1 and the compounds of Group A to Group C in the light emitting layer among the organic material layers, the organic light emitting device has more superior light emission efficiency and lifetime.

In one embodiment of the present application, the heterocyclic compounds according to Group A to Group C may be used as a P-type host.

In addition, the organic material layer includes one or more light emitting layers, and the light emitting layer includes the heterocyclic compound represented by Chemical Formula 1 and further includes one of the compounds of Group A to Group C. When including the heterocyclic compound represented by Chemical Formula 1 and one of the compounds of Group A to Group C at the same time in the light emitting layer among the organic material layers, the organic light emitting device has more superior light emission efficiency and lifetime due to an exciplex phenomenon.

In the organic light emitting device of the present application, the organic material layer includes a light emitting layer, and the light emitting layer may include the heterocyclic compound as a host material of a light emitting material.

In the organic light emitting device of the present application, the light emitting layer may include two or more host materials, and at least one of the host materials may include the heterocyclic compound as a host material of a light emitting material.

In the organic light emitting device of the present application, two or more host materials may be pre-mixed and used as the light emitting layer, and at least one of the two or more host materials may include the heterocyclic compound as a host material of a light emitting material.

The pre-mixing means placing and mixing two or more host materials of the light emitting layer in one source of supply before depositing on the organic material layer.

In the organic light emitting device of the present application, the light emitting layer may include two or more host materials, and the two or more host materials may each include one or more p-type host materials and n-type host materials, and at least one of the host materials may include the heterocyclic compound as a host material of a light emitting material. In this case, the organic light emitting device may have superior driving, efficiency and lifetime.

The organic light emitting device of the present disclosure may further include one or more layers selected from the group consisting of a light emitting layer, a hole injection layer, a hole transfer layer, an electron injection layer, an electron transfer layer, a hole auxiliary layer and a hole blocking layer.

The organic light emitting device according to one embodiment of the present application may be manufactured using common organic light emitting device manufacturing methods and materials except that the organic material layer is formed using the heterocyclic compound described above.

In addition, another embodiment of the present application provides a composition for an organic material layer of an organic light emitting device, the composition including the heterocyclic compound represented by Chemical Formula 1 and one of the compounds of Group A to Group C at the same time.

In another embodiment of the present application, the heterocyclic compound represented by Chemical Formula 1: one of the compounds of Group A to Group C may have a weight ratio of 1:10 to 10:1, 1:8 to 8:1, 1:5 to 5:1 or 1:2 to 2:1 in the composition, however, the weight ratio is not limited thereto.

Specific descriptions on the heterocyclic compound represented by Chemical Formula 1 and the compounds of Group A to Group C that the composition for an organic material layer includes are the same as the descriptions provided above.

FIG. 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

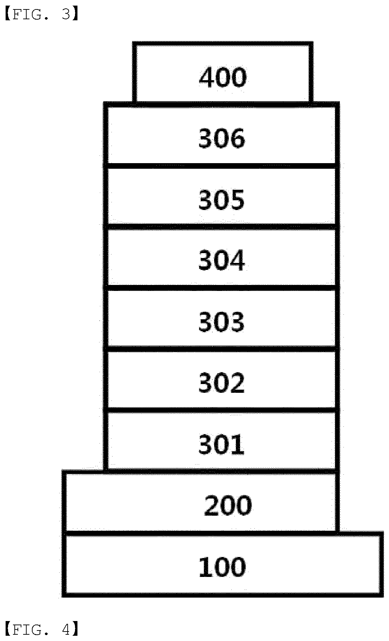

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

In addition, the organic light emitting device according to one embodiment of the present application includes a first electrode; a first stack provided on the first electrode and including a first light emitting layer; a charge generation layer provided on the first stack; a second stack provided on the charge generation layer and including a second light emitting layer; and a second electrode provided on the second stack.

Herein, the charge generation layer may include the heterocyclic compound represented by Chemical Formula 1. When the heterocyclic compound is used in the charge generation layer, driving, efficiency and lifetime of the organic light emitting device may be superior.

In addition, the first stack and the second stack may each independently further include one or more types of the hole injection layer, the hole transfer layer, the hole blocking layer, the electron transfer layer, the electron injection layer and the like described above.

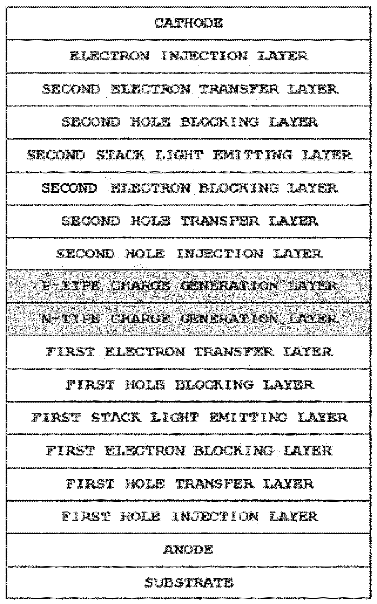

As the organic light emitting device according to one embodiment of the present application, an organic light emitting device having a 2-stack tandem structure is schematically illustrated in FIG. 4.

Herein, the first electron blocking layer, the first hole blocking layer, the second hole blocking layer and the like described in FIG. 4 may not be included in some cases.

In the organic light emitting device according to one embodiment of the present application, materials other than the heterocyclic compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

The organic light emitting device according to one embodiment of the present application may be a top-emission type, a bottom-emission type or a dual-emission type depending on the materials used.

The heterocyclic compound according to one embodiment of the present application may also be used in an organic electronic device including an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Preparation of Target Compound 1

### (1) Preparation of Compound F1

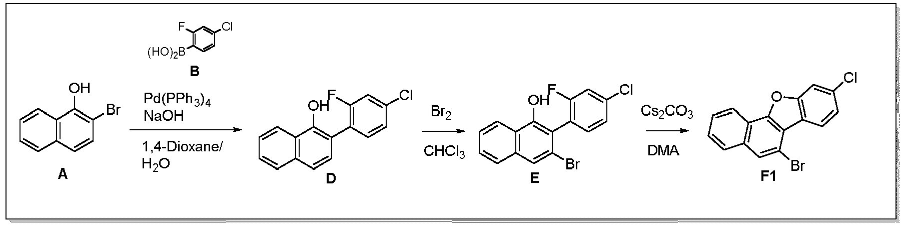

### 1) Preparation of Compound D

Compound A (20 g, 89.68 mmol), Compound B (15.6 g, 89.68 mmol), Pd(PPh₃)₄ (5.1 g, 4.48 mmol) and NaOH (7.2 g, 179.36 mmol) were dissolved in 1,4-dioxane/H₂O (200 mL/50 mL), and stirred for 8 hours at 100°C. The mixture solution completed with the reaction was dissolved in methylene chloride (MC), and extracted with water. The organic layer was dried with anhydrous MgSO₄, and then silica gel filtered. The solvent of the filtered filtrate was removed using a rotary evaporator to obtain yellow oil Compound D (11.7 g) in a 48% yield.

### 2) Preparation of Compound E

Compound D (11.7 g, 44.05 mmol) was dissolved in CHCl₃ (150 mL), and after adding Br₂ (1.2 mL, 44.05 mmol) dropwise thereto at 0°C, the mixture was stirred for 2 hours at room temperature. Methanol (MeOH) was introduced to the mixture solution completed with the reaction, and the result was stirred for 30 minutes and then filtered to obtain white solid Compound E (14.3 g) in a 93% yield.

### 3) Preparation of Compound F1

Compound E (14.3 g, 40.89 mmol) was dissolved in dimethylacetamide (DMA) (150 mL), and after introducing Cs₂CO₃ (26.5 g, 81.79 mmol) thereto, the mixture was stirred for 3 hours at 160°C. The mixture solution completed with the reaction was filtered, and the solvent of the filtrate was removed using a rotary evaporator to obtain white solid Compound F1 (11.5 g) in a 85% yield.

### (2) Preparation of Target Compound 1

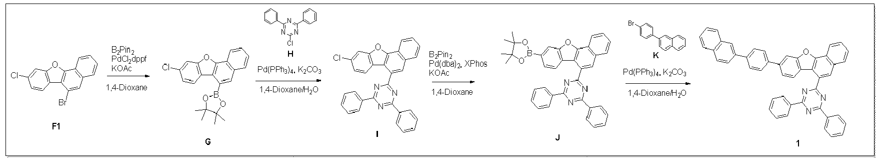

### 1) Preparation of Compound G

Compound F1 (20 g, 60.31 mmol), bis(pinacolato)diboron (30.6 g, 120.63 mmol), PdCl₂dppf (2.2 g, 3.02 mmol) and KOAc (18 g, 180.93 mmol) were dissolved in 1,4-dioxane (250 mL), and stirred for 4 hours at 100°C. The mixture solution completed with the reaction was concentrated, then dissolved in MC, and extracted with water. The organic layer was dried with anhydrous MgSO₄, and then silica gel filtered. The solvent of the filtered filtrate was removed using a rotary evaporator, and brown solid Compound G (20 g) was obtained in a crude state without separate preparation.

### 2) Preparation of Compound I

Compound G (20 g, 52.83 mmol), Compound H (14 g, 52.83 mmol), Pd(PPh₃)₄ (3 g, 2.64 mmol) and K₂CO₃ (14.5 g, 105.66 mmol) were dissolved in 1,4-dioxane/H₂O (250 mL/50 mL), and stirred for 6 hours at 100°C. After the reaction was completed, precipitated solids were filtered, and washed with water (H₂O), methanol (MeOH) and acetone to obtain white solid Compound I (13.8 g) in a 54% yield.

### 3) Preparation of Compound J

Compound I (13.8 g, 28.53 mmol), bis(pinacolato)diboron (14.5 g, 57.06 mmol), Pd(dba)₂ (1.6 g, 2.85 mmol), XPhos (2.7 g, 5.71 mmol) and KOAc (8.4 g, 85.59 mmol) were dissolved in 1,4-dioxane (150 mL), and stirred for 14 hours at 100°C. The mixture solution completed with the reaction was concentrated, then dissolved in methylene chloride (MC), and extracted with water. The organic layer was dried with anhydrous MgSO₄, and then silica gel filtered. The solvent of the filtered filtrate was removed using a rotary evaporator, and brown solid Compound J (14 g) was obtained in a crude state without separate preparation.

### 4) Preparation of Target Compound 1

Compound J (14 g, 24.35 mmol), Compound K (6.9 g, 24.35 mmol), Pd(PPh₃)₄ (1.4 g, 1.22 mmol) and K₂CO₃ (6.7 g, 48.69 mmol) were dissolved in 1,4-dioxane/H₂O (200 mL/40 mL), and stirred for 5 hours at 100°C. After the reaction was completed, precipitated solids were filtered, and washed with H₂O, MeOH and acetone to obtain white solid target Compound 1 (K) (11.7 g) in a 74% yield.

Compound F1 of the following Table 1 was prepared in the same manner as in Preparation of Compound F1 except that Intermediate A and Intermediate B of the following Table 1 were used instead of Compounds A and B, respectively.

**[Table 1]**

| Intermediate A | Intermediate B | F1 |
|---|---|---|
| 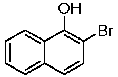 | 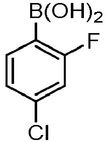 | 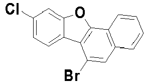 |
| 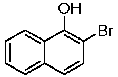 | 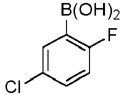 | 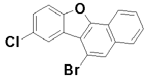 |

In addition, target compounds of the following Table 2 were prepared in the same manner as in Preparation of Target Compound 1 except that Intermediate F1, Intermediate H and Intermediate K of the following Table 2 were used instead of Compounds F1, H and K, respectively.

**[Table 2]**

| Compound No. | Intermediate F1 | Intermediate H | Intermediate K | Target Compound | Yield |
|---|---|---|---|---|---|
| 1 | 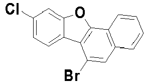 | 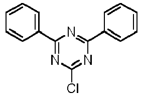 | 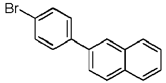 | 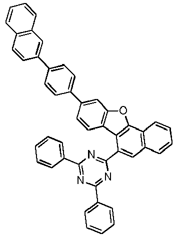 | 740 |
| 16 | 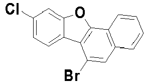 | 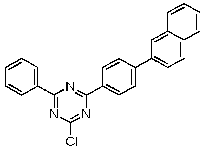 | 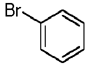 | 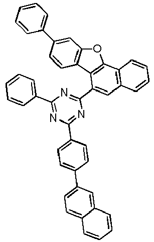 | 62% |
| 25 | 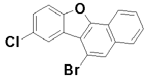 | 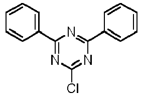 | 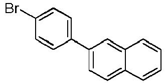 | 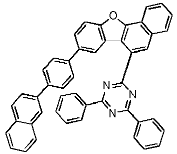 | 69% |
| 31 | 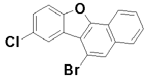 | 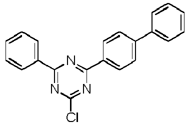 | 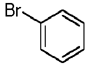 | 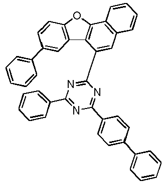 | 70% |
| 163 | 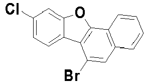 | 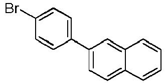 | 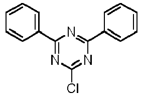 | 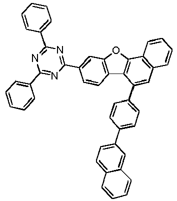 | 56% |
| 187 | 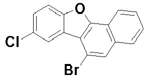 | 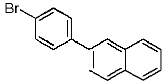 | 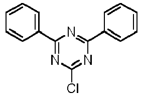 | 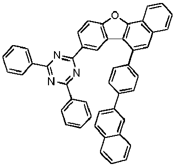 | 70% |
| 205 | 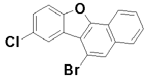 | 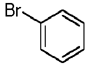 | 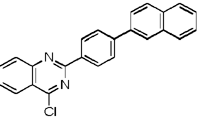 | 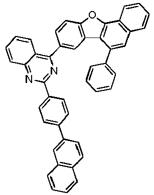 | 58% |
| 319 | 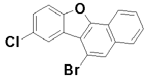 | 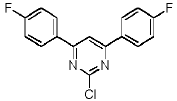 | 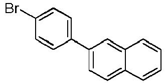 | 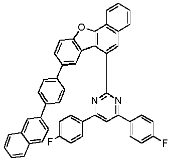 | 60% |

### <Preparation Example 2> Preparation of Target Compound 43

### (1) Preparation of Compound F2

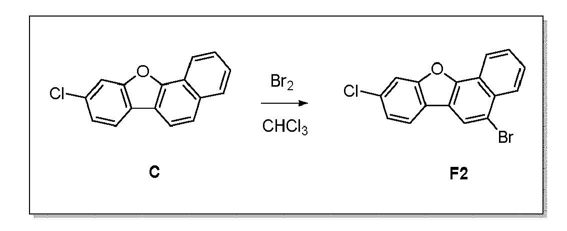

Compound C (10 g, 39.57 mmol) was dissolved in CHCl₃ (100 mL), and after adding Br₂ (2.04 mL, 39.57 mmol) dropwise thereto at 0°C, the mixture was stirred for 2 hours at room temperature. Methanol (MeOH) was introduced to the mixture solution completed with the reaction, and the result was stirred for 30 minutes and then filtered to obtain white solid Compound F2 (11.8 g) in a 90% yield.

Compound F2 of the following Table 3 was prepared in the same manner as in Preparation of Compound F2 except that Intermediate C of the following Table 3 was used instead of Compound C.

**[Table 3]**

| Intermediate C | F2 |
|---|---|
| 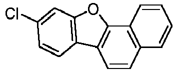 | 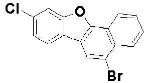 |
| 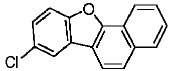 | 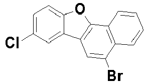 |
| 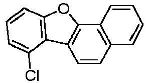 | 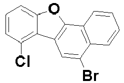 |

### (2) Preparation of Target Compound 43

Target Compound 43 (13.7 g) was obtained in a 59% yield in the same manner as in Preparation of Target Compound 1 of Preparation Example 1 except that Compound F2 was used instead of Compound F1.

In addition, target compounds of the following Table 4 were prepared in the same manner as in Preparation of Target Compound 43 except that Intermediate F2, Intermediate H and Intermediate K of the following Table 4 were used instead of Compounds F2, H and K, respectively.

**[Table 4]**

| Compound No. | Intermediate F2 | Intermediate H | Intermediate K | Target Compound | Yield |
|---|---|---|---|---|---|
| 43 | 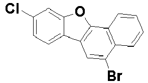 | 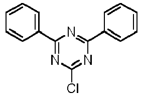 | 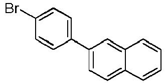 | 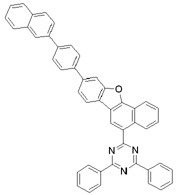 | 59% |
| 48 | 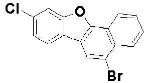 | 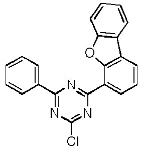 | 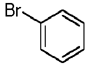 | 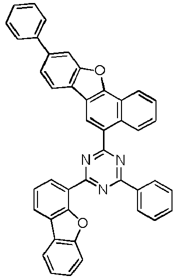 | 660 |
| 68 | 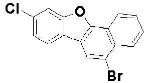 | 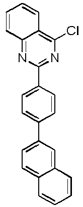 | 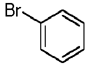 | 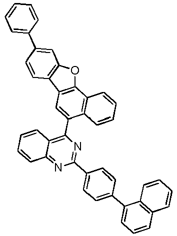 | 60% |
| 69 | 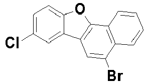 | 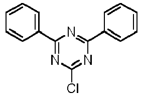 | 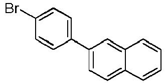 | 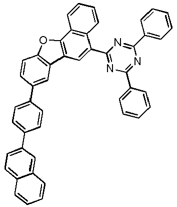 | 72% |
| 81 | 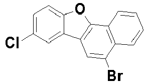 | 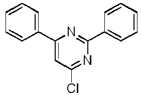 | 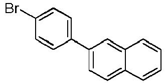 | 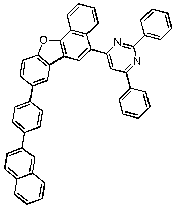 | 68% |
| 102 | 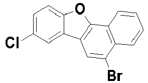 | 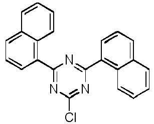 | 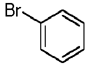 | 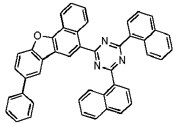 | 55% |
| 115 | 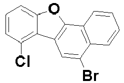 | 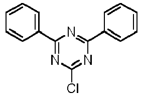 | 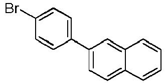 | 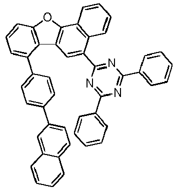 | 60% |
| 117 | 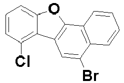 | 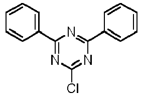 | 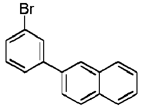 | 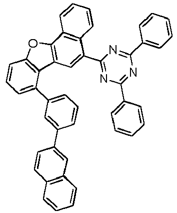 | 63% |
| 122 | 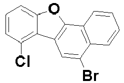 | 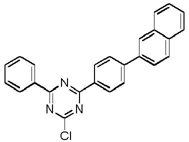 | 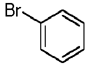 | 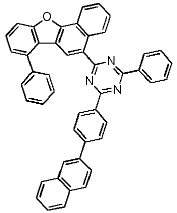 | 48% |
| 143 | 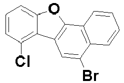 | 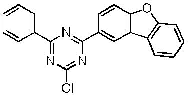 | 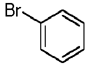 | 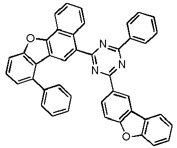 | 59% |
| 153 | 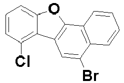 | 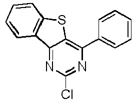 | 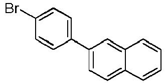 | 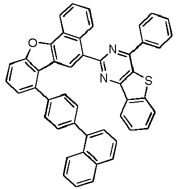 | 64% |
| 162 | 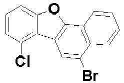 | 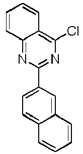 | 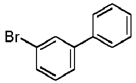 | 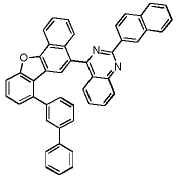 | 69% |
| 211 | 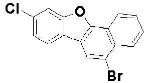 | 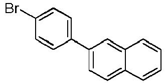 | 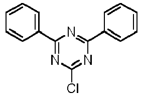 | 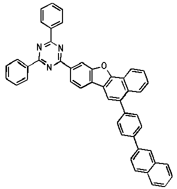 | 52% |
| 223 | 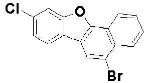 | 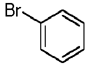 | 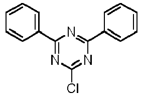 | 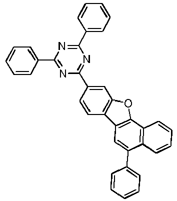 | 660 |
| 228 | 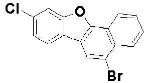 | 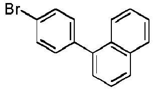 | 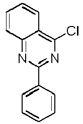 | 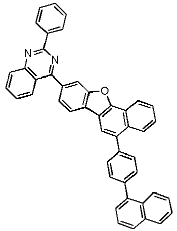 | 70% |
| 232 | 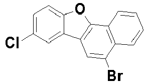 | 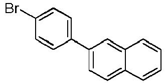 | 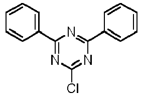 | 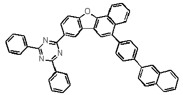 | 63% |
| 243 | 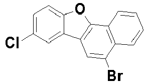 | 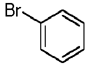 | 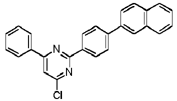 | 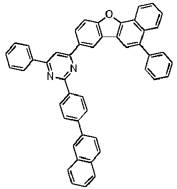 | 51% |
| 246 | 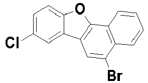 | 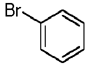 | 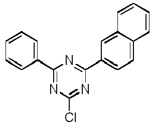 | 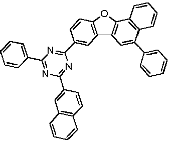 | 65% |
| 253 | 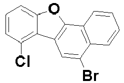 | 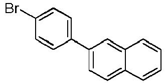 | 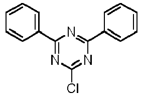 | 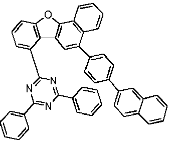 | 55% |
| 255 | 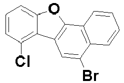 | 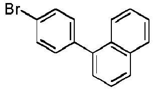 | 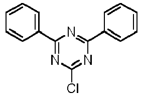 | 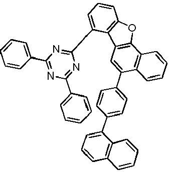 | 70% |
| 263 | 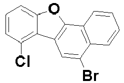 | 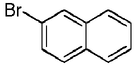 | 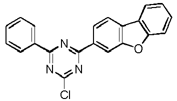 | 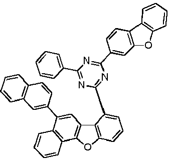 | 610 |
| 266 | 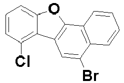 | 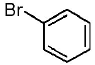 | 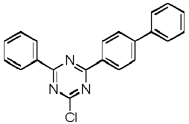 | 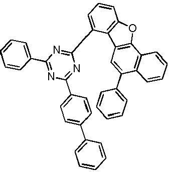 | 660 |
| 295 | 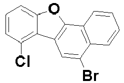 | 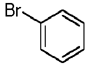 | 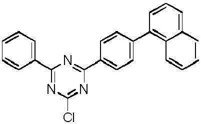 | 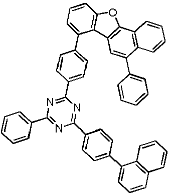 | 52% |
| 306 | 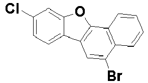 | 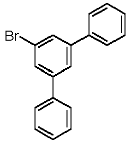 | 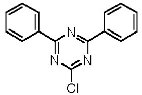 | 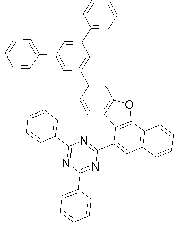 | 40% |

Compounds other than the compounds of the preparation examples were all synthesized in the same manner as in Preparation Examples 1 and 2, and the synthesis identification results are shown in the following Table 5 and Table 6. The following Table 5 shows measurement values of FD-mass spectrometry (FD-MS: field desorption mass spectrometry), and the following Table 6 shows measurement values of ¹H NMR (CDCl₃, 200 Mz).

**[Table 5]**

| Comp ound | FD-Mass | Comp ound | FD-Mass |
|---|---|---|---|
| 1 | m/z=651.75 (C47H29N3O=651.23) | 2 | m/z=651.75 (C47H29N3O=651.23) |
| 3 | m/z=651.75 (C47H29N3O=651.23) | 4 | m/z=651.75 (C47H29N3O=651.23) |
| 5 | m/z=651.75 (C47H29N3O=651.23) | 6 | m/z=651.75 (C47H29N3O=651.23) |
| 7 | m/z=625.72 (C45H27N3O=625.22) | 8 | m/z=651.75 (C47H29N3O=651.23) |
| 9 | m/z=651.75 (C47H29N3O=651.23) | 10 | m/z=651.75 (C47H29N3O=651.23) |
| 11 | m/z=651.75 (C47H29N3O=651.23) | 12 | m/z=601.69 (C43H27N3O=601.22) |
| 13 | m/z=691.77 (C49H29N3O2=691.23) | 14 | m/z=631.73 (C43H23N3OS=631.27) |
| 15 | m/z=615.68 (C43H25N3O2=615.19) | 16 | m/z=651.75 (C47H29N3O=651.23) |
| 17 | m/z=727.85 (C53H33N3O=727.26) | 18 | m/z=665.75 (C47H27N3O2=665.21) |
| 19 | m/z=651.75 (C47H29N3O=651.23) | 20 | m/z=623.73 (C46H28N2O=623.22) |
| 21 | m/z=638.70 (C46H26N2O2=638.20) | 22 | m/z=680.81 (C48H28N2OS=680.19) |
| 23 | m/z=726.86 (C54H34N2O=726.27) | 24 | m/z=664.75 (C48H28N2O2=664.22) |
| 25 | m/z=651.75 (C47H29N3O=651.23) | 26 | m/z=651.75 (C47H29N3O=651.23) |
| 27 | m/z=727.85 (C53H33N3O=727.26) | 28 | m/z=701.81 (C51H31N3O=701.25) |
| 29 | m/z=651.75 (C47H29N3O=651.23) | 30 | m/z=631.73 (C43H23N3OS=631.27) |
| 31 | m/z=601.69 (C43H27N3O=601.22) | 32 | m/z=615.68 (C43H25N3O2=615.19) |
| 33 | m/z=651.75 (C47H29N3O=651.23) | 34 | m/z=651.75 (C47H29N3O=651.23) |
| 35 | m/z=651.75 (C47H29N3O=651.23) | 36 | m/z=651.75 (C47H29N3O=651.23) |
| 37 | m/z=727.85 (C53H33N3O=727.26) | 38 | m/z=651.75 (C47H29N3O=651.23) |
| 39 | m/z=638.70 (C46H26N2O2=638.20) | 40 | m/z=634.73 (C46H28N2O=624.22) |
| 41 | m/z=680.81 (C48H28N2OS=680.19) | 42 | m/z=726.86 (C54H34N2O=726.27) |
| 43 | m/z=651.75 (C47H29N3O=651.23) | 44 | m/z=651.75 (C47H29N3O=651.23) |
| 45 | m/z=651.75 (C47H29N3O=651.23) | 46 | m/z=651.75 (C47H29N3O=651.23) |
| 47 | m/z=651.75 (C47H29N3O=651.23) | 48 | m/z=615.68 (C43H25N3O2=615.19) |
| 49 | m/z=615.68 (C43H25N3O2=615.19) | 50 | m/z=651.75 (C47H29N3O=651.23) |
| 51 | m/z=691.77 (C49H29N3O2=691.23) | 52 | m/z=651.75 (C47H29N3O=651.23) |
| 53 | m/z=727.85 (C53H33N3O=727.26) | 54 | m/z=625.72 (C45H27N3O=625.22) |
| 55 | m/z=651.75 (C47H29N3O=651.23) | 56 | m/z=651.75 (C47H29N3O=651.23) |
| 57 | m/z=638.70 (C46H26N2O2=638.20) | 58 | m/z=624.73 (C46H28N2O=624.22) |
| 59 | m/z=638.70 (C46H26N2O2=638.20) | 60 | m/z=624.73 (C46H28N2O=624.22) |
| 61 | m/z=680.81 (C48H28N2OS=680.19) | 62 | m/z=720.83 (C50H28N2OS=720.19) |
| 63 | m/z=664.75 (C78H28N2O2=664.22) | 64 | m/z=716.87 (C53H36N2O=716.18) |
| 65 | m/z=664.75 (C48H28N2O2=664.22) | 66 | m/z=680.81 (C48H28N2OS=680.19) |
| 67 | m/z=624.73 (C46H28N2O=624.22) | 68 | m/z=624.73 (C46H28N2O=624.22) |
| 69 | m/z=651.75 (C47H29N3O=651.23) | 70 | m/z=651.75 (C47H29N3O=651.23) |
| 71 | m/z=651.75 (C47H29N3O=651.23) | 72 | m/z=651.75 (C47H29N3O=651.23) |
| 73 | m/z=615.68 (C43H25N3O2=615.19) | 74 | m/z=665.75 (C47H27N3O2=665.21) |
| 75 | m/z=615.68 (C43H25N3O2=615.19) | 76 | m/z=665.75 (C47H27N3O2=665.21) |
| 77 | m/z=631.73 (C43H23N3OS=631.27) | 78 | m/z=615.68 (C43H25N3O2=615.19) |
| 79 | m/z=651.75 (C47H29N3O=651.23) | 80 | m/z=651.75 (C47H29N3O=651.23) |
| 81 | m/z=650.76 (C48H30N2O=650.24) | 82 | m/z=650.76 (C48H30N2O=650.24) |
| 83 | m/z=650.76 (C48H30N2O=650.24) | 84 | m/z=650.76 (C48H30N2O=650.24) |
| 85 | m/z=614.69 (C44H26N2O2=614.20) | 86 | m/z=614.69 (C44H26N2O2=614.20) |
| 87 | m/z=624.73 (C46H28N2O=624.22) | 88 | m/z=624.73 (C46H28N2O=624.22) |
| 89 | m/z=624.73 (C46H28N2O=624.22) | 90 | m/z=624.73 (C46H28N2O=624.22) |
| 91 | m/z=638.70 (C46H26N2O2=638.20) | 92 | m/z=638.70 (C46H26N2O2=638.20) |
| 93 | m/z=701.81 (C51H31N3O=701.25) | 94 | m/z=651.75 (C47H29N3O=651.23) |
| 95 | m/z=701.81 (C51H31N3O=701.25) | 96 | m/z=641.76 (C46H31N3O=641.25) |
| 97 | m/z=601.69 (C43H27N3=601.22) | 98 | m/z=651.75 (C47H29N3O=651.23) |
| 99 | m/z=701.81 (C51H31N3O=701.25) | 100 | m/z=651.75 (C47H29N3O=651.23) |
| 101 | m/z=651.75 (C47H29N3O=651.23) | 102 | m/z=625.72 (C45H27N3O=625.22) |
| 103 | m/z=624.73 (C46H28N2O=624.22) | 104 | m/z=624.73 (C46H28N2O=624.22) |
| 105 | m/z=638.70 (C46H26N2O2=638.20) | 106 | m/z=638.70 (C46H26N2O2=638.20) |
| 107 | m/z=680.81 (C48H28N2OS=680.19) | 108 | m/z=680.81 (C48H28N2OS=680.19) |
| 109 | m/z=664.75 (C48H28N2O2=664.22) | 110 | m/z=664.75 (C48H28N2O2=664.22) |
| 111 | m/z=604.72 (C42H24N2OS=604.16) | 112 | m/z=680.81 (C48H28N2OS=680.19) |
| 113 | m/z=680.81 (C48H28N2OS=680.19) | 114 | m/z=644.74 (C44H48N2O2S=644.16) |
| 115 | m/z=651.75 (C47H29N3O=651.23) | 116 | m/z=651.75 (C47H29N3O=651.23) |
| 117 | m/z=651.75 (C47H29N3O=651.23) | 118 | m/z=651.75 (C47H29N3O=651.23) |
| 119 | m/z=651.75 (C47H29N3O=651.23) | 120 | m/z=701.81 (C51H31N3O=701.25) |
| 121 | m/z=651.75 (C47H29N3O=651.23) | 122 | m/z=651.75 (C47H29N3O=651.23) |
| 123 | m/z=651.75 (C47H29N3O=651.23) | 124 | m/z=701.81 (C51H31N3O=701.25) |
| 125 | m/z=651.75 (C47H29N3O=651.23) | 126 | m/z=575.66 (C41H25N3O=575.20) |
| 127 | m/z=650.76 (C48H30N2O=650.24) | 128 | m/z=525.60 (C37H23N3O=525.18) |
| 129 | m/z=615.68 (C43H25N3O2=615.19) | 130 | m/z=650.76 (C48H30N2O=650.24) |
| 131 | m/z=650.76 (C48H30N2O=650.24) | 132 | m/z=650.76 (C48H30N2O=650.24) |
| 133 | m/z=701.81 (C51H31N3O=701.25) | 134 | m/z=651.75 (C47H29N3O=651.23) |
| 135 | m/z=701.81 (C51H31N3O=701.25) | 136 | m/z=641.76 (C46H31N3O=641.25) |
| 137 | m/z=651.75 (C47H29N3O=651.23) | 138 | m/z=651.75 (C47H29N3O=651.23) |
| 139 | m/z=651.75 (C47H29N3O=651.23) | 140 | m/z=625.72 (C45H27N3O=625.22) |
| 141 | m/z=638.70 (C46H26N2O2=638.20) | 142 | m/z=638.70 (C46H26N2O2=638.20) |
| 143 | m/z=638.70 (C46H26N2O2=638.20) | 144 | m/z=638.70 (C46H26N2O2=638.20) |
| 145 | m/z=665.75 (C47H27N3O2=665.21) | 146 | m/z=665.75 (C47H27N3O2=665.21) |
| 147 | m/z=665.75 (C47H27N3O2=665.21) | 148 | m/z=624.73 (C46H28N2O=624.22) |
| 149 | m/z=624.73 (C46H28N2O=624.22) | 150 | m/z=638.70 (C46H26N2O2=638.20) |
| 151 | m/z=638.70 (C46H26N2O2=638.20) | 152 | m/z=680.81 (C48H28N2OS=680.19) |
| 153 | m/z=680.81 (C48H28N2OS=680.19) | 154 | m/z=664.75 (C48H28N2O2=664.22) |
| 155 | m/z=604.72 (C42H24N2OS=604.16) | 156 | m/z=680.81 (C48H28N2OS=680.19) |
| 157 | m/z=680.81 (C48H28N2OS=680.19) | 158 | m/z=644.74 (C44H48N2O2S=644.16) |
| 159 | m/z=624.73 (C46H28N2O=624.22) | 160 | m/z=624.73 (C46H28N2O=624.22) |
| 161 | m/z=624.73 (C46H28N2O=624.22) | 162 | m/z=624.73 (C46H28N2O=624.22) |
| 163 | m/z=651.75 (C47H29N3O=651.23) | 164 | m/z=651.75 (C47H29N3O=651.23) |
| 165 | m/z=651.75 (C47H29N3O=651.23) | 166 | m/z=651.75 (C47H29N3O=651.23) |
| 167 | m/z=615.68 (C43H25N3O2=615.19) | 168 | m/z=615.68 (C43H25N3O2=615.19) |
| 169 | m/z=651.75 (C47H29N3O=651.23) | 170 | m/z=651.75 (C47H29N3O=651.23) |
| 171 | m/z=651.75 (C47H29N3O=651.23) | 172 | m/z=615.68 (C43H25N3O2=615.19) |
| 173 | m/z=615.68 (C43H25N3O2=615.19) | 174 | m/z=665.75 (C47H27N3O2=665.21) |
| 175 | m/z=615.68 (C43H25N3O2=615.19) | 176 | m/z=650.76 (C48H30N2O=650.24) |
| 177 | m/z=650.76 (C48H30N2O=650.24) | 178 | m/z=525.60 (C37H23N3O=525.18) |
| 179 | m/z=575.66 (C41H25N3O=575.20) | 180 | m/z=575.66 (C41H25N3O=575.20) |
| 181 | m/z=624.73 (C46H28N2O=624.22) | 182 | m/z=624.73 (C46H28N2O=624.22) |
| 183 | m/z=624.73 (C46H28N2O=624.22) | 184 | m/z=624.73 (C46H28N2O=624.22) |
| 185 | m/z=664.75 (C48H28N2O2=664.22) | 186 | m/z=680.81 (C48H28N2OS=680.19) |
| 187 | m/z=651.75 (C47H29N3O=651.23) | 188 | m/z=651.75 (C47H29N3O=651.23) |
| 189 | m/z=651.75 (C47H29N3O=651.23) | 190 | m/z=651.75 (C47H29N3O=651.23) |
| 191 | m/z=651.75 (C47H29N3O=651.23) | 192 | m/z=651.75 (C47H29N3O=651.23) |
| 193 | m/z=615.68 (C43H25N3O2=615.19) | 194 | m/z=615.68 (C43H25N3O2=615.19) |
| 195 | m/z=615.68 (C43H25N3O2=615.19) | 196 | m/z=615.68 (C43H25N3O2=615.19) |
| 197 | m/z=650.76 (C48H30N2O=650.24) | 198 | m/z=650.76 (C48H30N2O=650.24) |
| 199 | m/z=601.69 (C43H27N3=601.22) | 200 | m/z=525.60 (C37H23N3O=525.18) |
| 201 | m/z=575.66 (C41H25N3O=575.20) | 202 | m/z=624.73 (C46H28N2O=624.22) |
| 203 | m/z=624.73 (C46H28N2O=624.22) | 204 | m/z=624.73 (C46H28N2O=624.22) |
| 205 | m/z=624.73 (C46H28N2O=624.22) | 206 | m/z=680.81 (C48H28N2OS=680.19) |
| 207 | m/z=631.73 (C43H23N3OS=631.27) | 208 | m/z=638.70 (C46H26N2O2=638.20) |
| 209 | m/z=624.73 (C46H28N2O=624.22) | 210 | m/z=651.75 (C47H29N3O=651.23) |
| 211 | m/z=651.75 (C47H29N3O=651.23) | 212 | m/z=651.75 (C47H29N3O=651.23) |
| 213 | m/z=651.75 (C47H29N3O=651.23) | 214 | m/z=651.75 (C47H29N3O=651.23) |
| 215 | m/z=651.75 (C47H29N3O=651.23) | 216 | m/z=651.75 (C47H29N3O=651.23) |
| 217 | m/z=615.68 (C43H25N3O2=615.19) | 218 | m/z=615.68 (C43H25N3O2=615.19) |
| 219 | m/z=615.68 (C43H25N3O2=615.19) | 220 | m/z=615.68 (C43H25N3O2=615.19) |
| 221 | m/z=650.76 (C48H30N2O=650.24) | 222 | m/z=650.76 (C48H30N2O=650.24) |
| 223 | m/z=525.60 (C37H23N3O=525.18) | 224 | m/z=601.69 (C43H27N3=601.22) |
| 225 | m/z=575.66 (C41H25N3O=575.20) | 226 | m/z=624.73 (C46H28N2O=624.22) |
| 227 | m/z=624.73 (C46H28N2O=624.22) | 228 | m/z=624.73 (C46H28N2O=624.22) |
| 229 | m/z=624.73 (C46H28N2O=624.22) | 230 | m/z=680.81 (C48H28N2OS=680.19) |
| 231 | m/z=680.81 (C48H28N2OS=680.19) | 232 | m/z=651.75 (C47H29N3O=651.23) |
| 233 | m/z=651.75 (C47H29N3O=651.23) | 234 | m/z=651.75 (C47H29N3O=651.23) |
| 235 | m/z=651.75 (C47H29N3O=651.23) | 236 | m/z=651.75 (C47H29N3O=651.23) |
| 237 | m/z=651.75 (C47H29N3O=651.23) | 238 | m/z=651.75 (C47H29N3O=651.23) |
| 239 | m/z=615.68 (C43H25N3O2=615.19) | 240 | m/z=615.68 (C43H25N3O2=615.19) |
| 241 | m/z=615.68 (C43H25N3O2=615.19) | 242 | m/z=615.68 (C43H25N3O2=615.19) |
| 243 | m/z=650.76 (C48H30N2O=650.24) | 244 | m/z=650.76 (C48H30N2O=650.24) |
| 245 | m/z=601.69 (C43H27N3=601.22) | 246 | m/z=575.66 (C41H25N3O=575.20) |
| 247 | m/z=575.66 (C41H25N3O=575.20) | 248 | m/z=624.73 (C46H28N2O=624.22) |
| 249 | m/z=624.73 (C46H28N2O=624.22) | 250 | m/z=624.73 (C46H28N2O=624.22) |
| 251 | m/z=624.73 (C46H28N2O=624.22) | 252 | m/z=680.81 (C48H28N2OS=680.19) |
| 253 | m/z=680.81 (C48H28N2OS=680.19) | 254 | m/z=651.75 (C47H29N3O=651.23) |
| 255 | m/z=651.75 (C47H29N3O=651.23) | 256 | m/z=651.75 (C47H29N3O=651.23) |
| 257 | m/z=651.75 (C47H29N3O=651.23) | 258 | m/z=651.75 (C47H29N3O=651.23) |
| 259 | m/z=651.75 (C47H29N3O=651.23) | 260 | m/z=615.68 (C43H25N3O2=615.19) |
| 261 | m/z=615.68 (C43H25N3O2=615.19) | 262 | m/z=615.68 (C43H25N3O2=615.19) |
| 263 | m/z=615.68 (C43H25N3O2=615.19) | 264 | m/z=665.75 (C47H27N3O2=665.21) |
| 265 | m/z=665.75 (C47H27N3O2=665.21) | 266 | m/z=525.60 (C37H23N3O=525.18) |
| 267 | m/z=601.69 (C43H27N3=601.22) | 268 | m/z=575.66 (C41H25N3O=575.20) |
| 269 | m/z=575.66 (C41H25N3O=575.20) | 270 | m/z=650.76 (C48H30N2O=650.24) |
| 271 | m/z=650.76 (C48H30N2O=650.24) | 272 | m/z=650.76 (C48H30N2O=650.24) |
| 273 | m/z=650.76 (C48H30N2O=650.24) | 274 | m/z=624.73 (C46H28N2O=624.22) |
| 275 | m/z=624.73 (C46H28N2O=624.22) | 276 | m/z=624.73 (C46H28N2O=624.22) |
| 277 | m/z=624.73 (C46H28N2O=624.22) | 278 | m/z=588.65 (C42H24N2O2=588.18) |
| 279 | m/z=638.70 (C46H26N2O2=638.20) | 280 | m/z=588.65 (C42H24N2O2=588.18) |
| 281 | m/z=638.70 (C46H26N2O2=638.20) | 282 | m/z=624.73 (C46H28N2O=624.22) |
| 283 | m/z=624.73 (C46H28N2O=624.22) | 284 | m/z=624.73 (C46H28N2O=624.22) |
| 285 | m/z=680.81 (C48H28N2OS=680.19) | 286 | m/z=680.81 (C48H28N2OS=680.19) |
| 287 | m/z=680.81 (C48H28N2OS=680.19) | 288 | m/z=664.75 (C48H28N2O2=664.22) |
| 289 | m/z=604.72 (C42H24N2OS=604.16) | 290 | m/z=727.85 (C53H33N3O=727.26) |
| 291 | m/z=601.69 (C43H27N3=601.22) | 292 | m/z=651.75 (C47H29N3O=651.23) |
| 293 | m/z=727.85 (C53H33N3O=727.26) | 294 | m/z=727.85 (C53H33N3O=727.26) |
| 295 | m/z=726.86 (C54H34N2O=726.27) | 296 | m/z=727.85 (C53H33N3O=727.26) |
| 297 | m/z=727.85 (C53H33N3O=727.26) | 298 | m/z=677.79 (C49H31N3O=677.25) |
| 299 | m/z=727.85 (C53H33N3O=727.26) | 300 | m/z=561.58 (C37H21F2N3O=561.17) |
| 301 | m/z=593.65 (C41H24FN3O=593.16) | 302 | m/z=542.60 (C38H3FN2O=542.18) |
| 303 | m/z=687.73 (C47H27F2N3O=687.21) | 304 | m/z=669.74 (C47H28FN3O=669.22) |
| 305 | m/z=550.61 (C38H22N4O=550.18) | 306 | m/z=546.62 (C40H22N2O=546.17) |
| 307 | m/z=549.62 (C39H23N3O=549.18) | 308 | m/z=546.62 (C40H22N2O=546.17) |
| 309 | m/z=701.77 (C49H27N5O=701.22) | 310 | m/z=546.58 (C38H27N4O=546.15) |
| 311 | m/z=546.58 (C38H27N4O=546.15) | 312 | m/z=727.85 (C53H33N3O=727.26) |
| 313 | m/z=727.85 (C53H33N3O=727.26) | 314 | m/z=727.85 (C53H33N3O=727.26) |
| 315 | m/z=727.85 (C53H33N3O=727.26) | 316 | m/z=727.85 (C53H33N3O=727.26) |
| 317 | m/z=561.58 (C37H21F2N3O=561.17) | 318 | m/z=687.73 (C47H27F2N3O=687.21) |
| 319 | m/z=686.75 (C48H28F2N2O=686.22) | 320 | m/z=675.77 (C49H29N3O=675.23) |
| 321 | m/z=546.62 (C40H22N2O=546.17) | 322 | m/z=600.67 (C42H24N4O=600.20) |
| 323 | m/z=783.95 (C55H33N3OS=78317) | 324 | m/z=707.85 (C49H29N3OS=707.20) |
| 325 | m/z=750.90 (C56H34N2O=750.27) | 326 | m/z=767.89 (C55H33N3O2=767.26) |
| 327 | m/z=753.90 (C55H35N3O=753.28) | 328 | m/z=767.89 (C55H33N3O2=767.26) |

**[Table 6]**

| Example | ¹H NMR (CDCl₃, 200 Mz) |
|---|---|
| 1 | δ=9.25(d, 2H), 9.05(d, 4H), 8.68(d, 1H), 8.46(d, 1H), 8.26(d, 1H), 8.03(d, 1H), 7.95(s, 1H), 7.81∼7.69(m, 6H), 7.62∼7.53(m, 6H), 7.49∼7.33(m, 7H) |
| 16 | δ=8.75(d, 2H), 8.68~8.46(m, 5H), 8.40(t, 1H), 8.26(d, 1H), 8.11∼8.03(m, 4H), 7.95(s, 1H), 7.81∼7.69(m, 6H), 7.59∼7.53(m, 7H), 7.25(d, 2H) |
| 25 | δ=8.28(d, 4H), 8.00∼7.95(m, 5H), 7.81(d, 1H), 7.75∼7.72(m, 6H), 7.66(d, 1H), 7.51∼7.40(m, 7H), 7.33∼7.30(m, 5H) |
| 31 | δ=8.28(d, 4H), 8.00(d, 2H), 7.85∼7.81(m, 5H), 7.75∼7.72(m, 5H), 7.71(s, 1H), 7.66(d, 1H), 7.51∼7.40(m, 7H), 7.25(d, 2H) |
| 43 | δ=8.35(d, 2H), 8.15(d, 4H), 7.68(d, 1H), 7.56-7.50(m, 3H), 7.35(s, 1H), 7.32∼7.29(m, 6H), 7.22∼7.18(m, 6H), 7.09∼6.97(m, 5H) |
| 48 | δ=8.55(d, 2H), 8.25(m, 2H), 8.18(d, 1H), 7.98(d, 1H), 7.86-7.75(m, 6H), 7.71(s, 1H), 7.65(s, 1H), 7.52∼7.49(m, 6H), 7.22∼7.18(m, 5H) |
| 68 | δ=8.85(d, 2H), 8.45(d, 2H), 8.18~8.16(m, 2H), 7.98(d, 1H), 7.88-7.75(m, 10H), 7.71(s, 1H), 7.65(s, 1H), 7.52∼7.49(m, 7H), 7.25(d, 2H) |
| 69 | δ=8.65(d, 2H), 8.28∼8.26(m, 4H), 8.18(d, 1H), 8.06-7.92(m, 4H), 7.81(s, 1H), 7.71∼7.69(m, 6H), 7.59∼7.53(m, 7H), 7.25(m, 4H) |
| 81 | δ=8.57(d, 2H), 8.28~8.26(m, 4H), 8.23(s, 1H), 8.00-7.92(m, 4H), 7.81(s, 1H), 7.79∼7.71(m, 6H), 7.59~7.53(m, 7H), 7.25(m, 4H) |
| 102 | δ=8.28(d, 4H), 8.18(d, 2H), 8.08∼7.99(m, 5H), 7.75∼7.72(m, 5H), 7.71(s, 1H), 7.66(d, 1H), 7.51∼7.40(m, 7H), 7.25(d, 2H) |
| 115 | δ=8.57(d, 1H), 8.25(m, 4H), 8.18(d, 1H), 8.06∼8.03(m, 5H), 7.95(s, 1H), 7.75∼7.71(m, 4H), 7.62~7.53(m, 6H), 7.49∼7.43(m, 7H) |
| 117 | δ=8.57(d, 1H), 8.27(m, 4H), 8.15(d, 1H), 8.06∼7.92(m, 5H), 7.95(s, 1H), 7.75∼7.73(m, 4H), 7.71(s, 1H), 7.70(s, 1H), 7.62∼7.53(m, 6H), 7.50∼7.48(m, 5H) |
| 122 | δ=8.55(d, 1H), 8.27(m, 2H), 8.17(d, 1H), 7.92∼7.85(m, 8H), 7.71(s, 1H), 7.70(s, 1H), 7.62∼7.59(m, 7H), 7.43~7.39(m, 6H), 7.25(d, 2H) |
| 143 | δ=8.55(d, 2H), 8.25(m, 2H), 8.15(d, 1H), 7.80-7.73(m, 8H), 7.71(s, 1H), 7.52∼7.49(m, 6H), 7.242∼7.32(m, 5H) |
| 153 | δ=8.60(d, 1H), 8.42(d, 1H), 8.17(d, 1H), 8.06∼7.85(m, 4H), 7.79∼7.75(m, 3H), 7.72(s, 1H), 7.62∼7.59(m, 17H) |
| 162 | δ=9.09(s, 1H), 8.47∼8.41(m, 2H), 8.17∼8.15(m, 2H), 8.00∼7.92(m, |
| | 4H), 7.72∼7.69(m, 7H), 7.53∼7.44(m, 12H) |
| 163 | δ=8.26(m, 4H), 8.03∼7.89(m, 6H), 7.71∼7.59(m, 6H), 7.52∼7.49(m, 6H), 7.40∼7.37(m, 6H), 7.35(s, 1H) |
| 187 | δ=8.27(m, 4H), 8.00∼7.89(m, 5H), 7.81(d, 1H), 7.71∼7.68(m, 6H), 7.60∼7.52(m, 6H), 7.49∼7.40(m, 6H), 7.37(s, 1H) |
| 205 | δ=8.17(d, 1H), 8.02∼7.88(m, 6H), 7.85∼7.78(m, 6H), 7.60∼7.59(m, 3H), 7.58(s, 1H), 7.50∼7.45(m, 8H), 7.37(s, 1H), 7.25(d, 2H) |
| 211 | δ=8.58(d, 1H), 8.29(m, 4H), 8.18(d, 1H), 8.01∼7.92(m, 6H), 7.75∼7.73(m, 2H), 7.70(s, 1H), 7.60∼7.59(m, 3H), 7.50∼7.45(m, 6H), 7.37(s, 1H), 7.25(m, 4H) |
| 223 | δ=8.57(d, 1H), 8.38(m, 4H), 8.19(d, 1H), 7.95(d, 1H), 7.85∼7.78(m, 6H), 7.70(s, 1H), 7.64(s, 1H), 7.60∼7.59(m, 3H), 7.58(s, 1H), 7.50∼7.45(m, 4H) |
| 228 | δ=8.56(d, 2H), 8.42(d, 1H), 8.29(m, 2H), 8.18∼8.15(m, 2H), 7.95∼7.88(m, 7H), 7.80∼7.79(m, 3H), 7.70(s, 1H), 7.65∼7.59(m, 8H), 7.50∼7.45(m, 2H) |
| 232 | δ=8.59(d, 1H), 8.26(m, 4H), 8.19(d, 1H), 8.03∼7.89(m, 3H), 7.81(d, 1H), 7.75(s, 1H), 7.71(s, 1H), 7.66∼7.59(m, 9H), 7.52∼7.49(m, 3H), 7.25(m, 4H) |
| 243 | δ=8.55(d, 1H), 8.26(s, 1H), 8.19(d, 1H), 8.03∼7.89(m, 8H), 7.78(s, 1H), 7.72(s, 1H), 7.69∼7.55(m, 9H), 7.52∼7.49(m, 6H), 7.35(m, 2H) |
| 246 | δ=9.05(s, 1H), 8.56(d, 1H), 8.46(d, 1H), 8.29(d, 2H), 8.03∼7.96(m, 5H), 7.80∼7.79(m, 4H), 7.74(s, 1H), 7.70(s, 1H), 7.66∼7.51(m, 9H) |
| 253 | δ=8.59(d, 1H), 8.26(m, 4H), 8.20(d, 1H), 8.03∼7.89(m, 3H), 7.84(d, 1H), 7.75(s, 1H), 7.71(s, 1H), 7.66∼7.59(m, 9H), 7.50∼7.40(m, 4H), 7.30 (m, 4H) |
| 255 | δ=8.55(d, 1H), 8.42(d, 1H), 8.26(m, 4H), 8.20(d, 1H), 8.03∼8.00(m, 3H), 7.85(d, 1H), 7.75(s, 1H), 7.71(s, 1H), 7.66∼7.59(m, 12H), 7.30(m, 4H) |
| 263 | δ=8.58(d, 1H), 8.28(m, 2H), 8.19(d, 1H), 8.03∼7.89(m, 5H), 7.75(s, 1H), 7.64(s, 1H), 7.66∼7.59(m, 9H), 7.56(s, 1H), 7.50∼7.46(m, 6H) |
| 266 | δ=8.60(d, 1H), 8.26(m, 2H), 8.17(d, 1H), 8.01∼7.86(m, 5H), 7.73(s, 1H), 7.66∼7.59(m, 9H), 7.52∼7.49(m, 6H), 7.25(d, 2H) |
| 295 | δ=8.60(d, 1H), 8.55(d, 1H), 8.40(d, 1H), 8.26(m, 2H), 8.17(d, 1H), 8.08∼8.00(m, 3H), 7.93∼7.80(m, 5H), 7.73(s, 1H), 7.66∼7.59(m, 9H), 7.52∼7.49(m, 5H), 7.30(d, 2H), 7.25(d, 2H) |
| 306 | δ=8.53-8.50(m, 2H), 8.20(d, 1H), 8.01-7.99(m, 4H), 7.90∼7.86(m, 8H), 7.73(s, 1H), 7.68∼7.58(m, 5H), 7.52∼7.49(m, 8H), 7.47-7.40(m, 2H) |
| 319 | δ=8.26(s, 1H), 8.17∼8.15(m, 4H), 8.08~8.00(m, 3H), 7.93(d, 1H), 7.81(d, 1H), 7.73(s, 1H), 7.60∼7.55(m, 6H), 7.52∼7.49(m, 6H), 7.35(s, 1H), 7.25(m, 4H) |

### <Experimental Example 1>

### 1) Manufacture of Organic Light Emitting Device-Red Single Host

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer TAPC (4,4'-cyclohexylidenebis[N,N-bis(4-methylphenyl)benzenamine]), which are common layers, were formed.

A light emitting layer was thermal vacuum deposited thereon as follows. The light emitting layer was deposited to 500 Å by using compounds described in the following Table 5 as a red host and doping (piq)₂(Ir) (acac), a red phosphorescent dopant, to the host by 3 wt% based on a total weight of the light emitting layer. After that, bathocuproine (hereinafter, BCP) was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, organic light emitting devices of Comparative Examples 1 to 10 and Examples 1 to 16 were manufactured.

Meanwhile, all the organic compounds required to manufacture the OLED were vacuum sublimation purified under 10⁻⁸ torr to 10⁻⁶ torr for each material to be used in the OLED manufacture.

Compounds A to J used in Comparative Examples 1 to 10 are as follows.

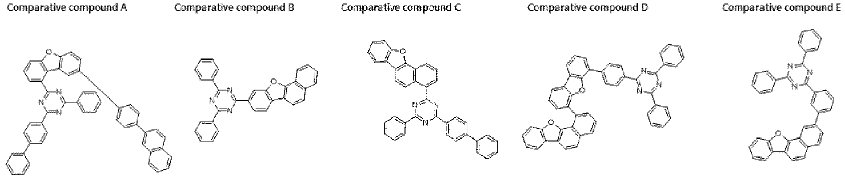

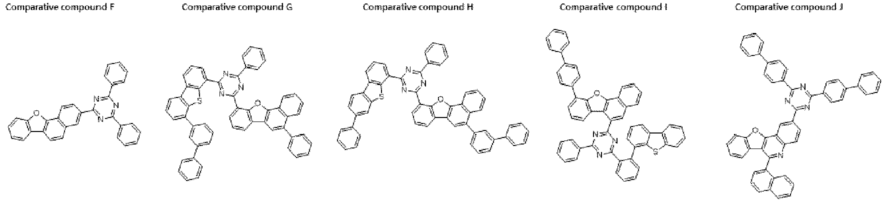

### 2) Driving Voltage and Light Emission Efficiency of Organic Light Emitting Device

For each of the organic light emitting devices of Comparative Examples 1 to 4 and Examples 1 to 16 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. The T₉₀ means a lifetime (unit: h, time), a time taken to become 90% with respect to initial luminance.

Measured properties of the organic light emitting devices are as shown in the following Table 7.

**[Table 7]**

| | Compound | Driving Voltage (V) | Efficiency (cd/A) | Color Coordinate (x, Y) | Lifetime (T₉₀) |
|---|---|---|---|---|---|
| Comparative Example 1 | Comparative Compound A | 10.35 | 8.5 | 0.672, 0.328 | - |
| Comparative Example 2 | Comparative Compound B | 9.74 | 8.0 | 0.676, 0.324 | 8 |
| Comparative Example 3 | Comparative Compound C | 9.50 | 6.7 | 0.680, 0.320 | 12 |
| Comparative Example 4 | Comparative Compound D | 9.70 | 6.2 | 0.677, 0.322 | 17 |
| Comparative Example 5 | Comparative Compound E | 9.81 | 7.0 | 0.681, 0.319 | 10 |
| Comparative Example 6 | Comparative Compound F | 10.04 | 7.5 | 0.678, 0.321 | 9 |
| Comparative Example 7 | Comparative Compound G | 10.21 | 6.0 | 0.685, 0.315 | 21 |
| Comparative Example 8 | Comparative Compound H | 9.91 | 6.5 | 0.692, 0.308 | 22 |
| Comparative Example 9 | Comparative Compound I | 10.01 | 5.8 | 0.689, 0.311 | 17 |
| Comparative Example 10 | Comparative Compound J | 12.34 | 5.5 | 0.678, 0.321 | 16 |
| Example 1 | 1 | 8.90 | 10.2 | 0.679, 0.321 | 20 |
| Example 2 | 16 | 8.88 | 13.5 | 0.684, 0.316 | 12 |
| Example 3 | 31 | 8.13 | 12.5 | 0.685, 0.314 | 12 |
| Example 4 | 48 | 8.90 | 11.6 | 0.679, 0.321 | 25 |
| Example 5 | 69 | 8.74 | 10.5 | 0.681, 0.319 | 11 |
| Example 6 | 81 | 9.01 | 11.4 | 0.692, 0.308 | 14 |
| Example 7 | 102 | 8.90 | 10.9 | 0.689, 0.311 | 19 |
| Example 8 | 115 | 8.03 | 12.0 | 0.681, 0.319 | 25 |
| Example 9 | 122 | 8.11 | 12.4 | 0.678, 0.321 | 22 |
| Example 10 | 153 | 8.28 | 12.5 | 0.685, 0.315 | 16 |
| Example 11 | 163 | 8.50 | 11.6 | 0.692, 0.308 | 10 |
| Example 12 | 187 | 8.90 | 12.3 | 0.689, 0.311 | 28 |
| Example 13 | 223 | 8.78 | 13.2 | 0.678, 0.321 | 25 |
| Example 14 | 228 | 9.00 | 11.0 | 0.679, 0.321 | 22 |
| Example 15 | 253 | 8.00 | 12.2 | 0.689, 0.311 | 31 |
| Example 16 | 266 | 7.95 | 12.0 | 0.678, 0.321 | 28 |

As seen from Table 7, using the compound corresponding to Chemical Formula 1 of the present application as a single host of a light emitting layer of an organic light emitting device as in Examples 1 to 16 resulted in enhanced results in terms of driving and efficiency of the device compared to Comparative Examples 1 to 4 using Comparative Compounds A to D not corresponding to Chemical Formula 1 of the present application as a single host of a light emitting layer of an organic light emitting device.

This is considered to be due to the fact that, when using a unipolar N-type compound like Comparative Compounds A to J as a single host in a light emitting layer of an organic light emitting device, high driving voltage and overall poor lifetime and efficiency properties were obtained since there was almost no hole injection ability by having a dibenzofuran linker.

On the other hand, it is considered that the compound corresponding to Chemical Formula 1 of the present application has T1 value and band gap suitable as a red host by introducing naphthobenzofuran with expanded π-conjugation to the dibenzofuran linker, which helps with enhancement in efficiency and driving voltage of the device. Herein, the T1 value means an energy level value in a triplet state.

In addition, it is considered that a structure that is more thermally stable and has enhanced electron mobility properties is obtained by specific positions of the naphtobenzofuran linker being substituted with substituents, which helps with enhancement in efficiency and driving voltage of the device.

In addition, compared to the comparative compounds having a simple dibenzofuran linker or having substituents formed in one direction, the compound corresponding to Chemical Formula 1 of the present application having substituents extending in both directions has advantages of having a fast electron transfer ability and being more stable to heat when used in a device due to more expanded conjugation compared to the comparative compounds.

In a compound having substituents at the same positions as Comparative Compound B, intermolecular interactions are inhibited due to strong steric hindrance and oxygen atom masking. When using a compound like Comparative Compound B in a device, this functions as one cause of packing structure defects in the compound, which may result in decreases in efficiency and lifetime of the device.

In addition, when substituents are positioned at the same positions as in Comparative Compounds A and C to J, HOMO/LUMO electron distribution clouds largely overlap compared to in the compound corresponding to Chemical Formula 1 of the present application, which increases a difference in the energy gap between S1 and T1, and an effect of increasing efficiency obtained by ISC (intersystem crossing) of excitons may not be expected.

### <Experimental Example 2>

### 1) Manufacture of Organic Light Emitting Device-Red N+P Mixed Host

Organic light emitting devices of Comparative Examples 11 and 12 and Examples 17 to 32 were manufactured in the same manner as in Experimental Example 1 except that, as the red host when forming the light emitting layer, the compound corresponding to Chemical Formula 1 of the present disclosure and a P-type host having strong hole transfer (HT) properties or a bipolar arylamine compound were mixed as described in the following Table 8.

The N+P mix means mixing the N-type host and the P-type host.

### 2) Driving Voltage and Light Emission Efficiency of Organic Light Emitting Device

For each of the organic light emitting devices of Comparative Examples 11 and 12 and Examples 17 to 32, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Measured properties of the organic light emitting devices are as shown in the following Table 8.

**[Table 8]**

| | Light Emitting Layer Compound | Ratio (N:P) | Driving Voltage (V) | Efficie ncy (cd/A) | Color Coordinate (x, Y) | Lifet ime (T₉₀) |
|---|---|---|---|---|---|---|
| Comparative Example 11 | C-4 | - | 4.52 | 19.5 | 0.681, 0.319 | 55 |
| Comparative Example 12 | C-21 | - | 4.72 | 19.0 | 0.675, 0.325 | 38 |
| Example 17 | A-1:Compound 31 | 1:1 | 4.10 | 17.1 | 0.674, 0.326 | 101 |
| Example 18 | A-3:Compound 102 | 1:1 | 4.60 | 17.0 | 0.673, 0.327 | 128 |
| Example 19 | A-4:Compound 16 | 1:1 | 4.31 | 19.5 | 0.682, 0.318 | 95 |
| Example 20 | A-5:Compound 115 | 1:1 | 4.02 | 18.5 | 0.677, 0.323 | 85 |
| Example 21 | A-6:Compound 81 | 1:1 | 4.72 | 16.4 | 0.675, 0.325 | 131 |
| Example 22 | B-1:Compound 266 | 1:1 | 4.85 | 16.0 | 0.681, 0.319 | 75 |
| Example 23 | B-2:Compound 228 | 1:1 | 5.00 | 17.2 | 0.675, 0.325 | 80 |
| Example 24 | B-3:Compound 31 | 1:1 | 4.78 | 16.3 | 0.680, 0.320 | 105 |
| Example 25 | B-4:Compound 153 | 1:1 | 5.08 | 17.1 | 0.684, 0.316 | 82 |
| Example 26 | C-4:Compound 43 | 1:1 | 4.00 | 20.0 | 0.685, 0.315 | 118 |
| Example 27 | C-21:Compound 102 | 1:1 | 4.50 | 19.3 | 0.676, 0.324 | 130 |
| Example 28 | C-29:Compound 223 | 1:1 | 4.05 | 21.1 | 0.682, 0.318 | 108 |
| Example 29 | C-31:Compound 81 | 1:1 | 4.45 | 19.0 | 0.673, 0.327 | 126 |
| Example 30 | C-40:Compound 228 | 1:1 | 4.43 | 21.0 | 0.679, 0.321 | 95 |
| Example 31 | C-50:Compound 48 | 1:1 | 4.42 | 21.1 | 0.677, 0.323 | 106 |
| Example 32 | C-54:Compound 115 | 1:1 | 4.00 | 19.7 | 0.684, 0.316 | 111 |

The P-type host or the bipolar arylamine compound of Table 8 was selected from among compounds of the following Groups A to C.

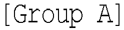

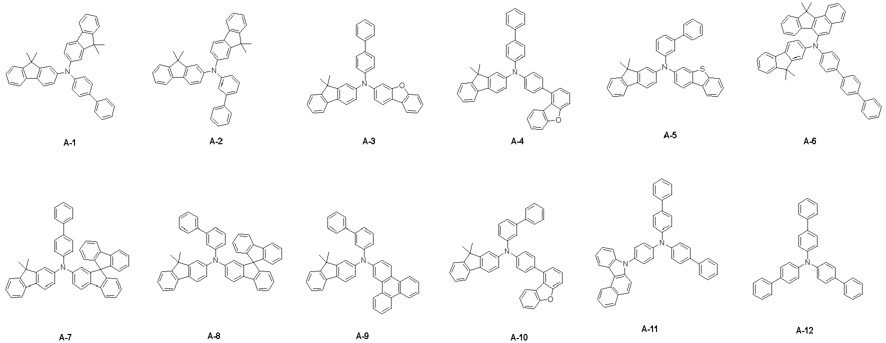

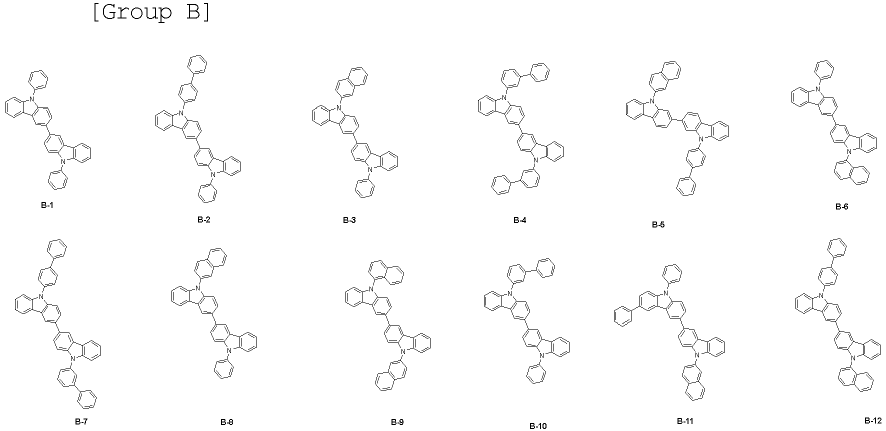

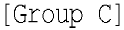

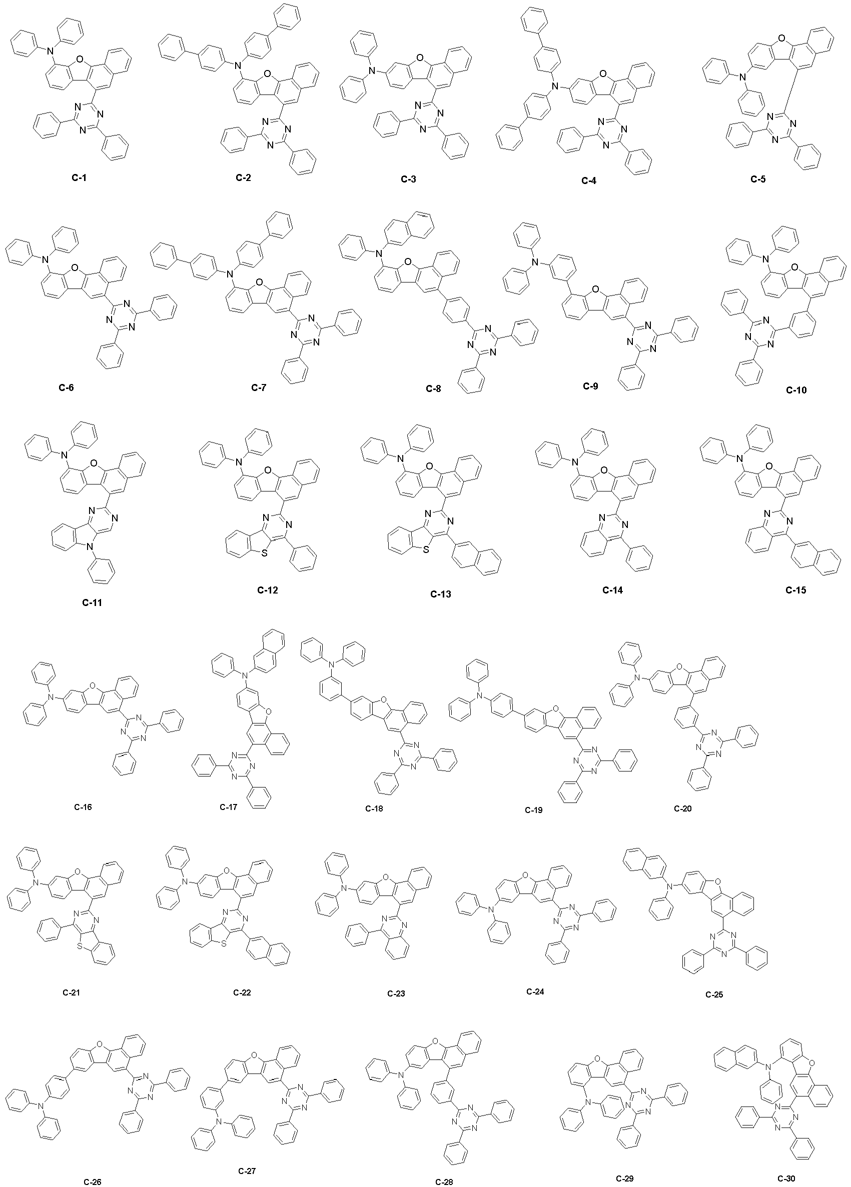

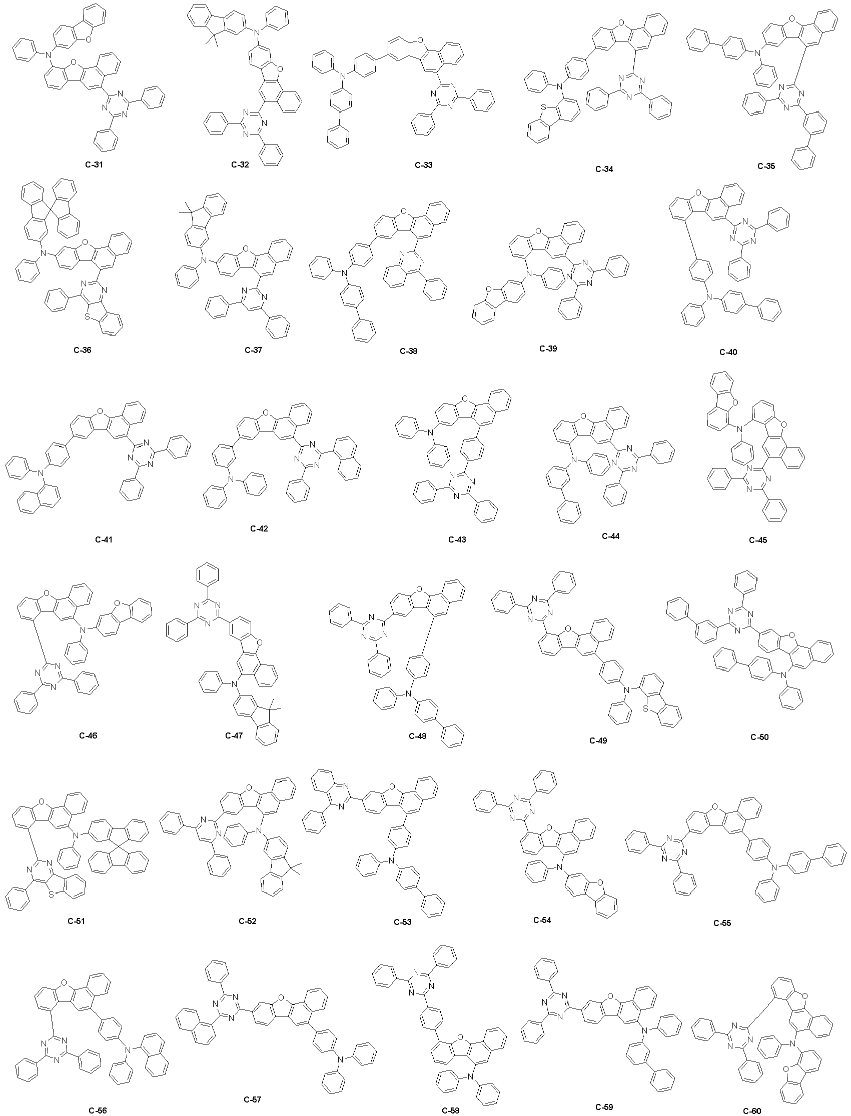

As seen from Table 8, it was identified that, when using the unipolar N-type compound having a naphthobenzofuran linker corresponding to Chemical Formula 1 of the present application mixed with the unipolar P-type compound or bipolar arylamine compound corresponding to Groups A to C as a host of a light emitting layer of an organic light emitting device as in Examples 17 to 32, excellent effects were obtained in the properties of efficiency and lifetime compared to when using the unipolar N-type compound having a naphthobenzofuran linker corresponding to Chemical Formula 1 of the present application as a single host.

This is considered to be due to the fact that, when mixing the unipolar N-type compound corresponding to Chemical Formula 1 of the present application and the unipolar P-type compound or bipolar arylamine compound, an exciplex phenomenon occurred due to strong hole transfer (HT) properties of the arylamine. In addition, it is considered that the result of excellent driving voltage was also obtained due to a low hole injection barrier.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%.

In general, an arylamine bipolar compound has strong hole transfer (HT) properties and electron transfer (ET) properties at the same time due to low hole and electron injection barriers of a bipolar compound, and thereby has narrow band gap and low T1 energy value, and accordingly, may also exhibit excellent efficiency in a phosphorescent red device as a single host. However, by having fast hole mobility, the lifetime tends to somewhat decrease due to deterioration in the device.

However, this was able to be resolved by mixing with a unipolar N-type compound having a naphthobenzofuran linker like Chemical Formula 1 of the present application. This means that a proper charge rate balance is obtained by mixing the two compounds in the light emitting layer, which reduces deterioration and expands the recombination zone, and as a result, an effect of increasing a lifetime is obtained without decreasing superior efficiency, an advantage of the arylamine bipolar compound.

### <Reference Numeral>

- 100:: Substrate
- 200:: Anode
- 300:: Organic Material Layer
- 301:: Hole Injection Layer
- 302:: Hole Transfer Layer
- 303:: Light Emitting Layer
- 304:: Hole Blocking Layer
- 305:: Electron Transfer Layer
- 306:: Electron Injection Layer
- 400:: Cathode

## Claims

1. A heterocyclic compound represented by the following Chemical Formula 1: 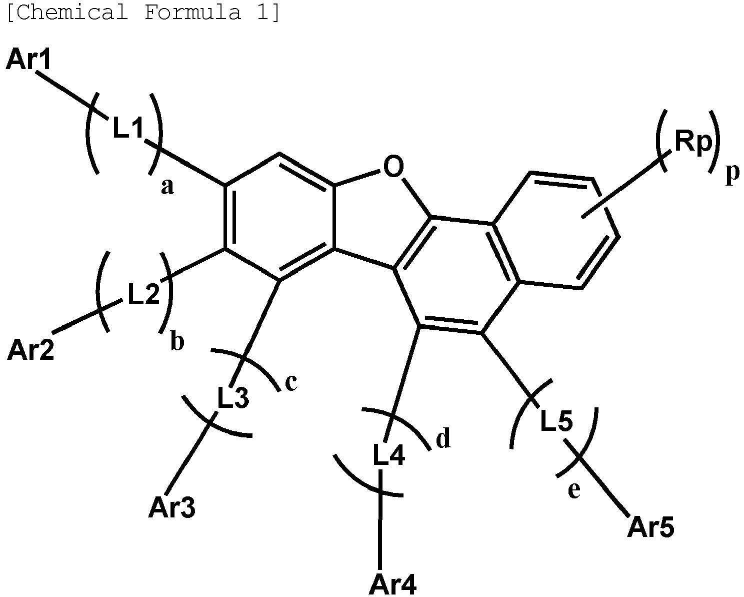 wherein, in Chemical Formula 1,
L1 to L5 are the same as or different from each other, and each independently a direct bond; a substituted or unsubstituted arylene group having 6 to 60 carbon atoms; or a substituted or unsubstituted heteroarylene group having 2 to 60 carbon atoms;
a, b, c, d and e are each an integer of 0 to 3, and when a, b, c, d and e are each 2 or greater, substituents in the parentheses are the same as or different from each other;
Ar1 to Ar5 are the same as or different from each other, and each independently a substituted or unsubstituted aryl group having 6 to 60 carbon atoms; or a substituted or unsubstituted heteroaryl group having 2 to 60 carbon atoms;
at least one of Ar1 to Ar5 is a monocyclic or polycyclic heteroaryl group having 2 to 60 carbon atoms substituted or unsubstituted and including one or more Ns; or an aryl group having 6 to 60 carbon atoms substituted with one or more cyano groups; and
Rp is hydrogen; deuterium; a halogen group; or a substituted or unsubstituted alkyl group having 6 to 60 carbon atoms, p is an integer of 0 to 4, and when p is 2 or greater, substituents in the parentheses are the same as or different from each other.

2. The heterocyclic compound of Claim 1, wherein the substituted or unsubstituted means being substituted with one or more substituents selected from the group consisting of deuterium; a cyano group; a halogen group; linear or branched alkyl having 1 to 60 carbon atoms; linear or branched alkenyl having 2 to 60 carbon atoms; linear or branched alkynyl having 2 to 60 carbon atoms; monocyclic or polycyclic cycloalkyl having 3 to 60 carbon atoms; monocyclic or polycyclic heterocycloalkyl having 2 to 60 carbon atoms; monocyclic or polycyclic aryl having 6 to 60 carbon atoms; monocyclic or polycyclic heteroaryl having 2 to 60 carbon atoms; -SiRR'R"; - P(=O)RR'; alkylamine having 1 to 20 carbon atoms; monocyclic or polycyclic arylamine having 6 to 60 carbon atoms; and monocyclic or polycyclic heteroarylamine having 2 to 60 carbon atoms, or being unsubstituted, or being substituted with a substituent linking two or more substituents selected from among the substituents illustrated above, and R, R' and R" are the same as or different from each other and each independently substituted or unsubstituted alkyl having 1 to 60 carbon atoms; substituted or unsubstituted aryl having 6 to 60 carbon atoms; or substituted or unsubstituted heteroaryl having 2 to 60 carbon atoms.

3. The heterocyclic compound of Claim 1, wherein Chemical Formula 1 is represented by the following Chemical Formula 1-1 or 1-2: 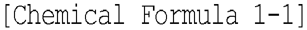 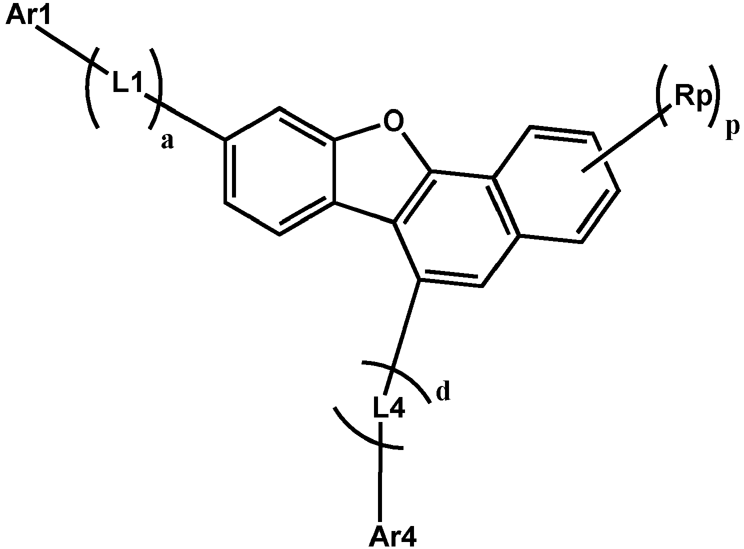 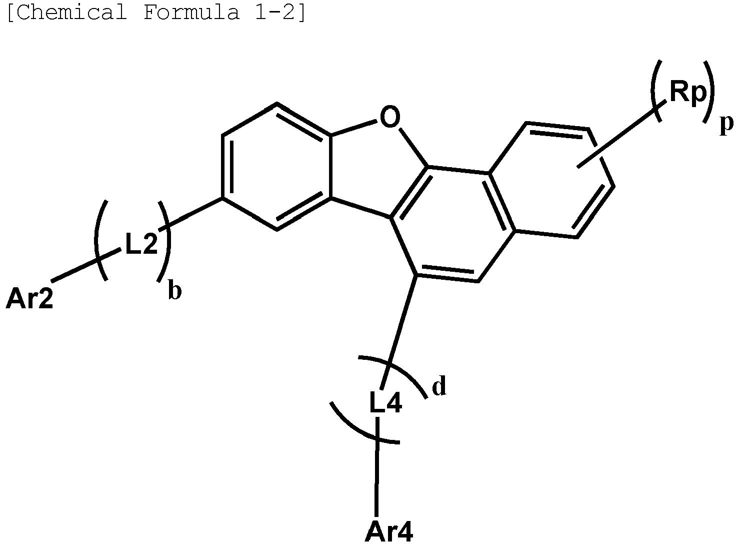
in Chemical Formula 1-1, Ar1 and Ar4 are the same as or different from each other and each independently a substituted or unsubstituted aryl group having 6 to 60 carbon atoms; or a substituted or unsubstituted heteroaryl group having 2 to 60 carbon atoms, at least one of Ar1 and Ar4 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1; and
in Chemical Formula 1-2, Ar2 and Ar4 are the same as or different from each other and each independently a substituted or unsubstituted aryl group having 6 to 60 carbon atoms; or a substituted or unsubstituted heteroaryl group having 2 to 60 carbon atoms, at least one of Ar2 and Ar4 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1.

4. The heterocyclic compound of Claim 1, wherein Chemical Formula 1 is represented by any one of the following Chemical Formulae 1-3 to 1-5: 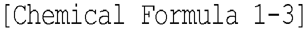 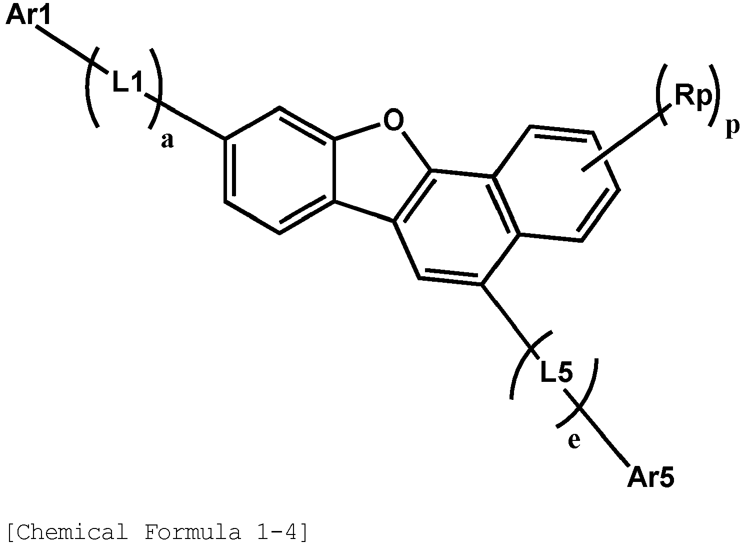 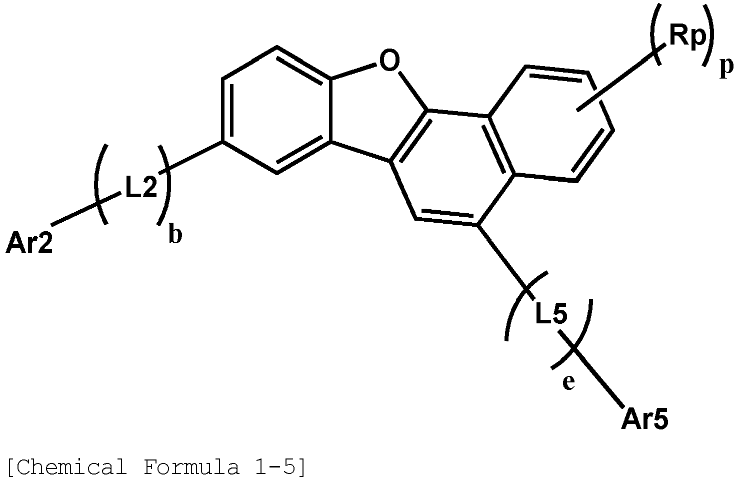 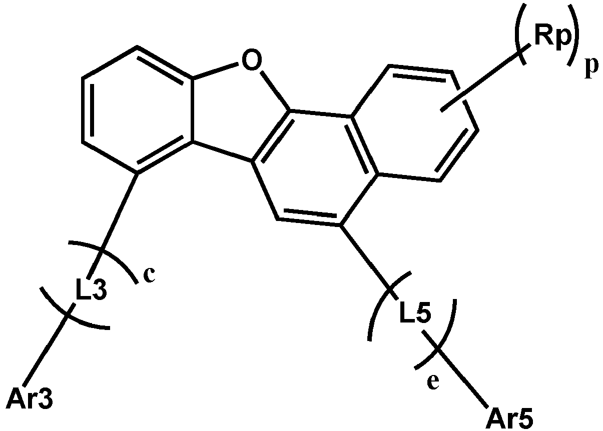
in Chemical Formula 1-3, Ar1 and Ar5 are the same as or different from each other and each independently a substituted or unsubstituted aryl group having 6 to 60 carbon atoms; or a substituted or unsubstituted heteroaryl group having 2 to 60 carbon atoms, at least one of Ar1 and Ar5 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1;
in Chemical Formula 1-4, Ar2 and Ar5 are the same as or different from each other and each independently a substituted or unsubstituted aryl group having 6 to 60 carbon atoms; or a substituted or unsubstituted heteroaryl group having 2 to 60 carbon atoms, at least one of Ar2 and Ar5 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1; and
in Chemical Formula 1-5, Ar3 and Ar5 are the same as or different from each other and each independently a substituted or unsubstituted aryl group having 6 to 60 carbon atoms; or a substituted or unsubstituted heteroaryl group having 2 to 60 carbon atoms, at least one of Ar3 and Ar5 is a monocyclic or polycyclic heteroaryl group having 2 to 60 carbon atoms substituted or unsubstituted and including one or more Ns; or an aryl group having 6 to 60 carbon atoms substituted with one or more cyano groups, and the rest have the same definitions as in Chemical Formula 1.

5. The heterocyclic compound of Claim 1, wherein the monocyclic or polycyclic heteroaryl group including one or more Ns is a group represented by the following Chemical Formula 3: 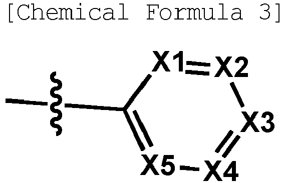 in Chemical Formula 3,
X1 is CR1 or N, X2 is CR2 or N, X3 is CR3 or N, X4 is CR4 or N, X5 is CR5 or N, and at least one of X1 to X5 is N; and
R1 to R5 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -P(=O)R10R12; and NR13R14, or two or more groups adjacent to each other bond to each other to form a substituted or unsubstituted aliphatic or aromatic hydrocarbon ring or heteroring, R10 and R12 to R14 are the same as or different from each other and each independently hydrogen; deuterium; a halogen group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms, and 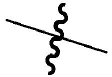 is a site linked to Chemical Formula 1.

6. The heterocyclic compound of Claim 1, wherein Chemical Formula 1 is represented by any one of the following compounds:

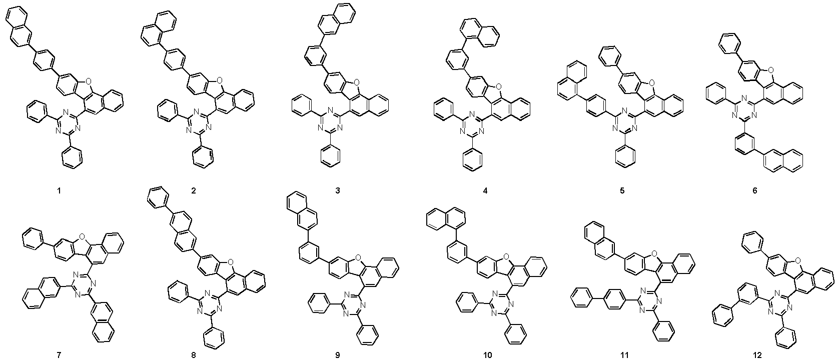

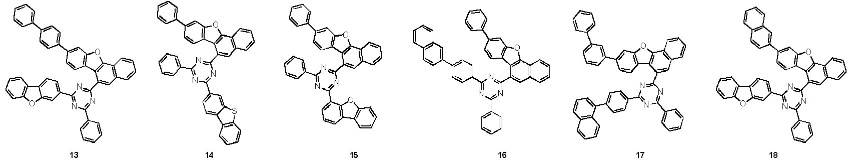

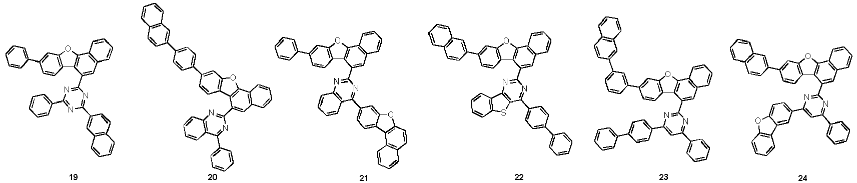

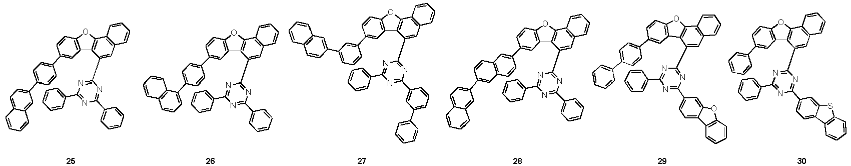

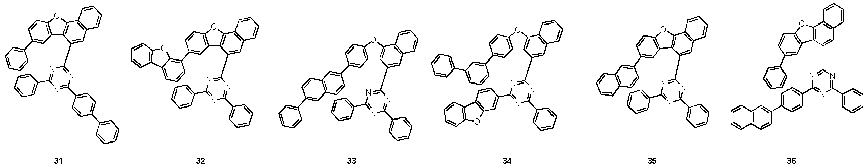

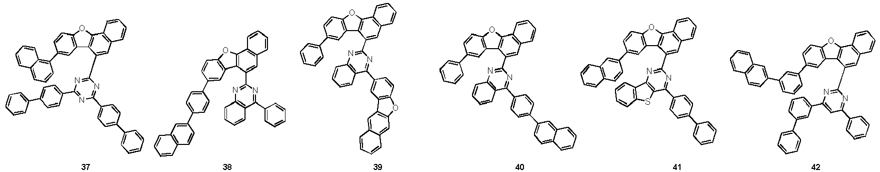

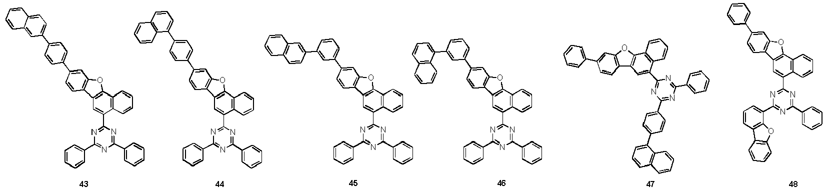

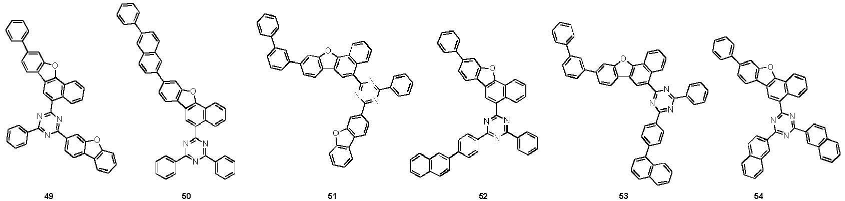

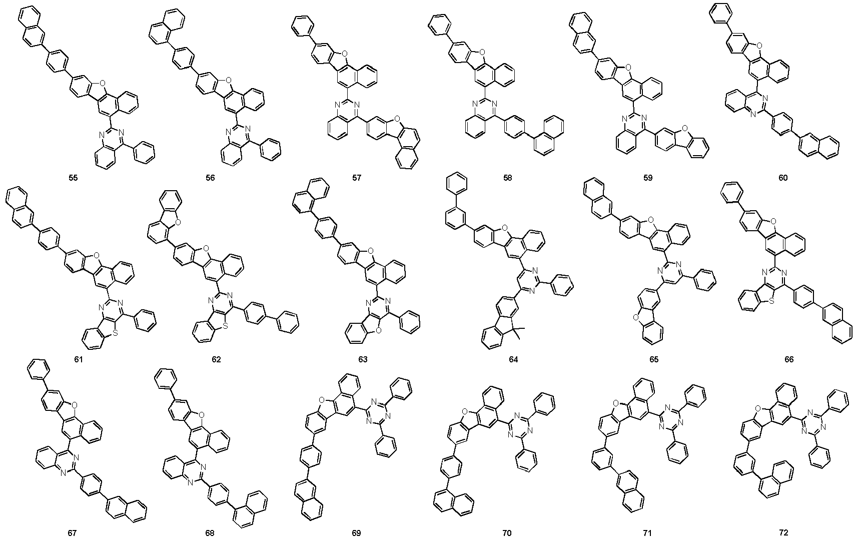

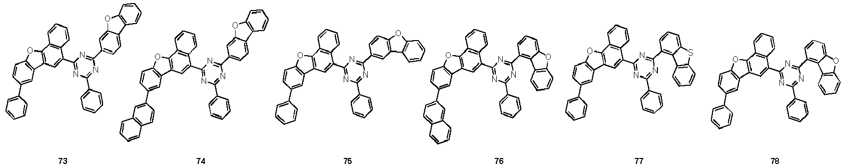

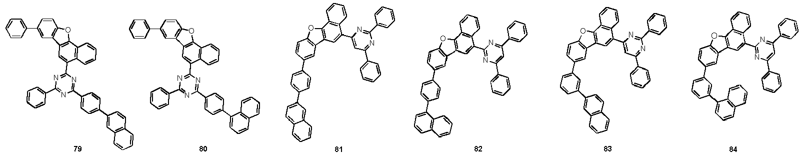

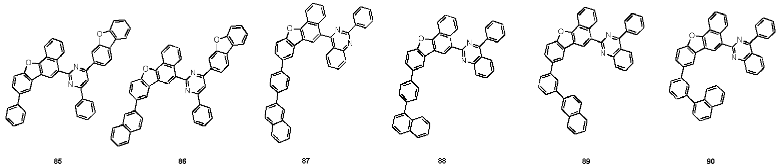

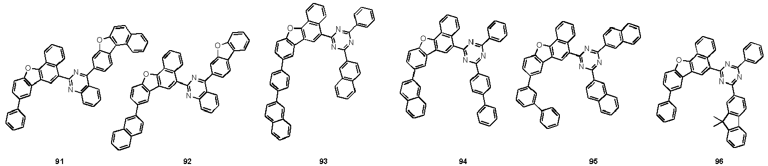

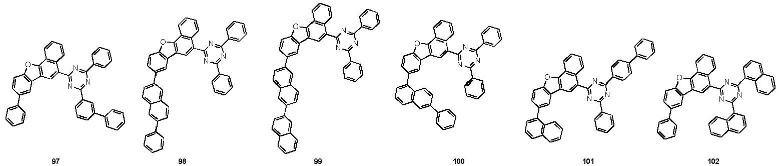

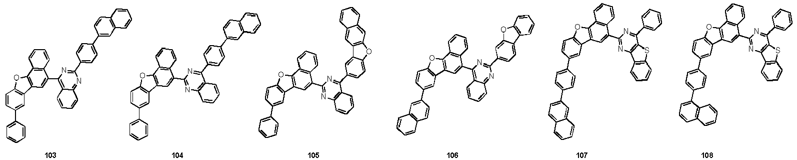

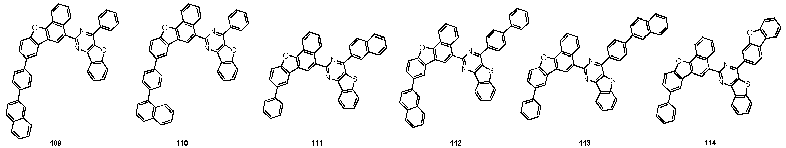

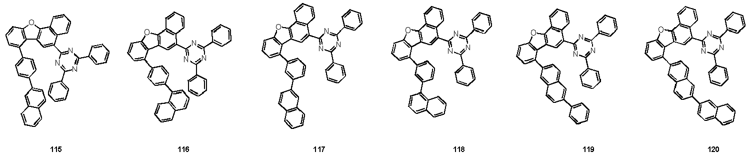

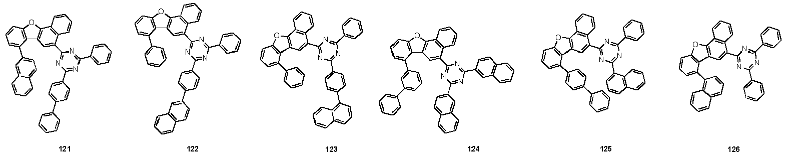

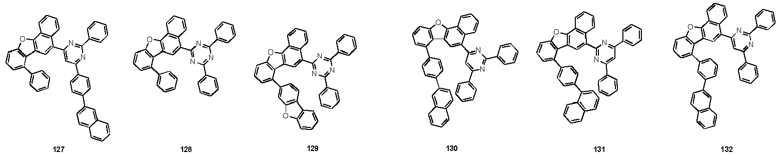

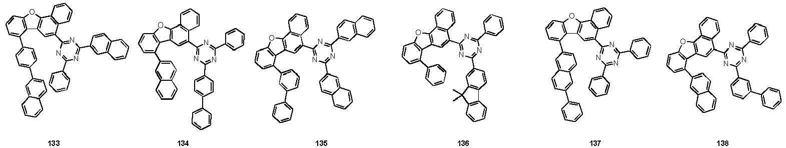

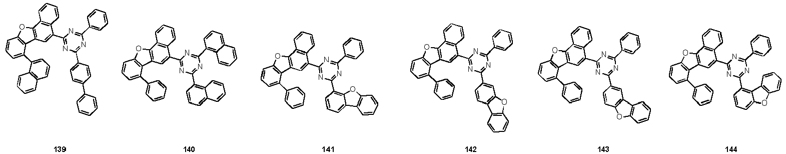

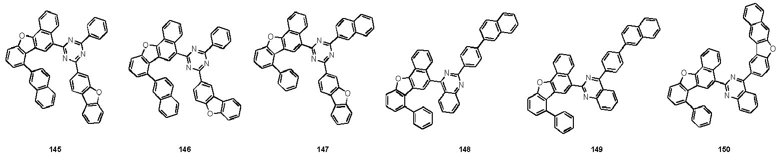

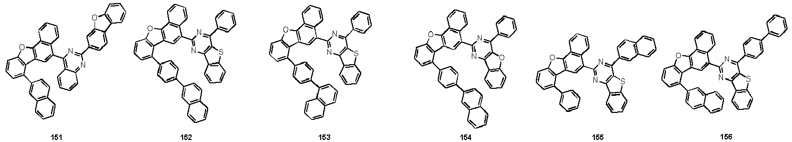

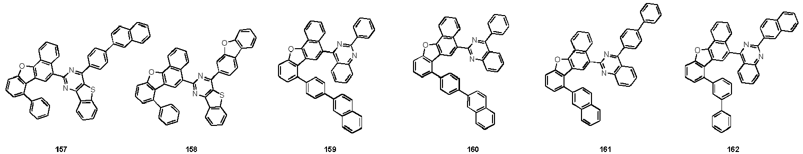

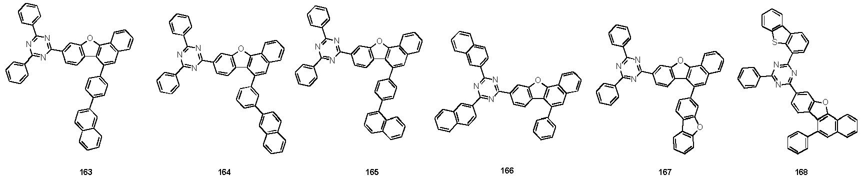

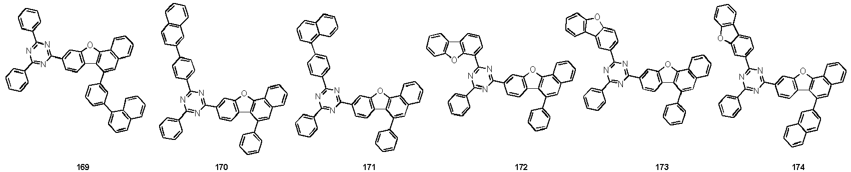

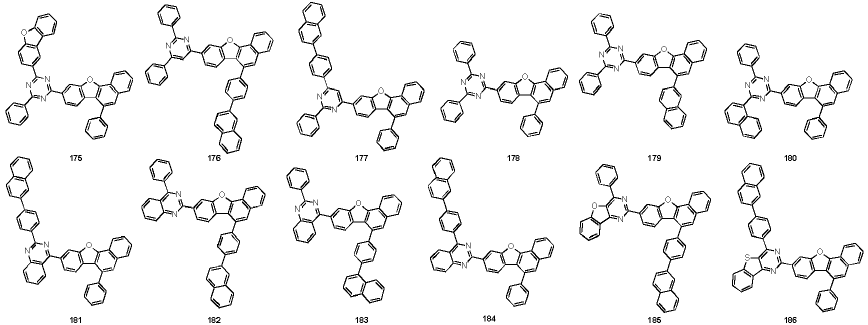

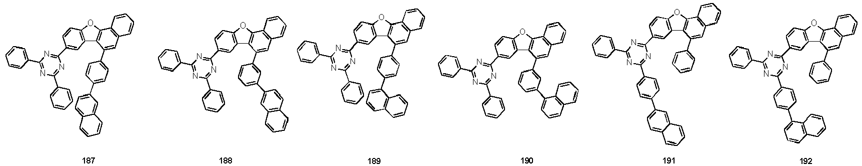

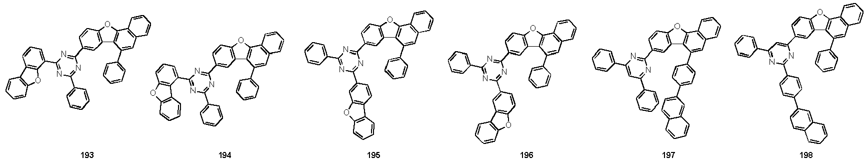

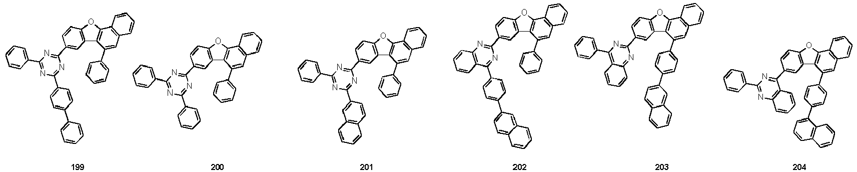

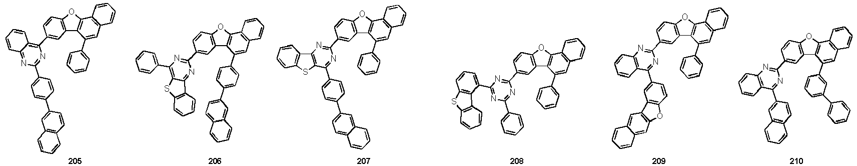

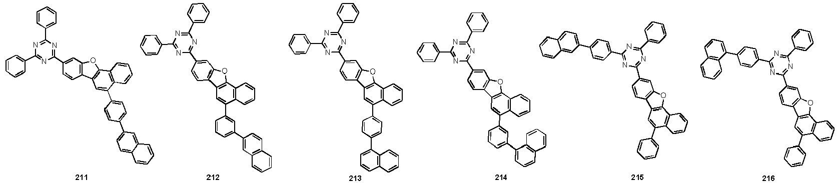

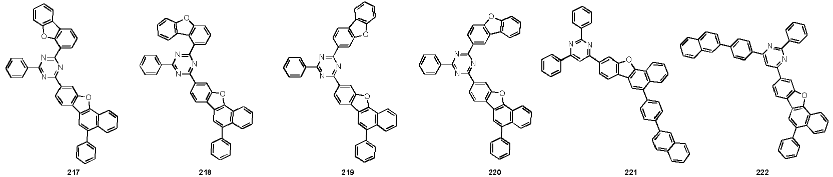

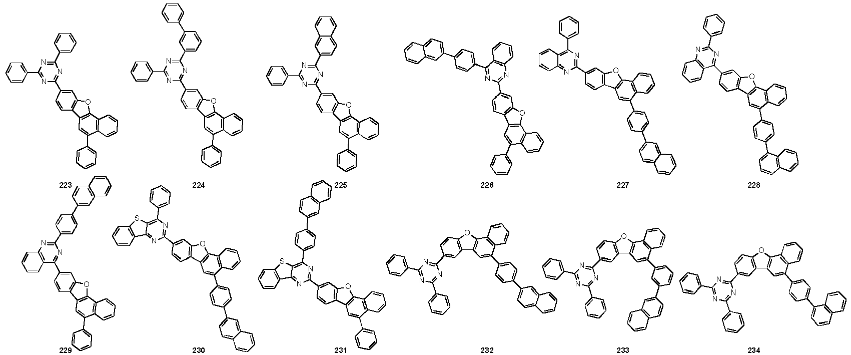

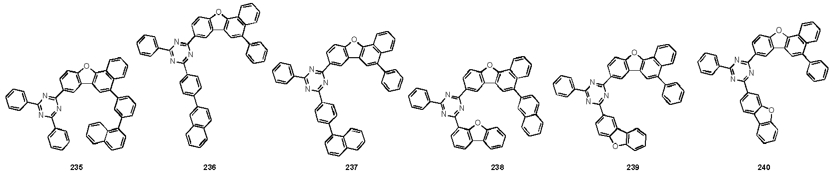

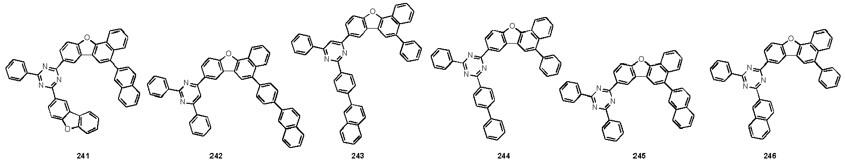

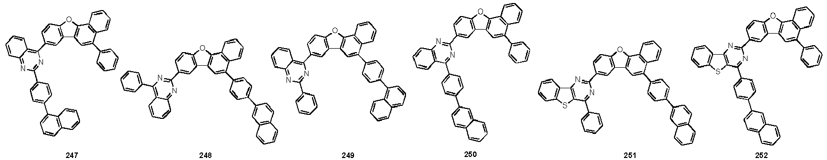

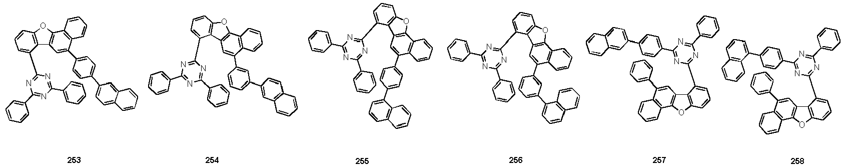

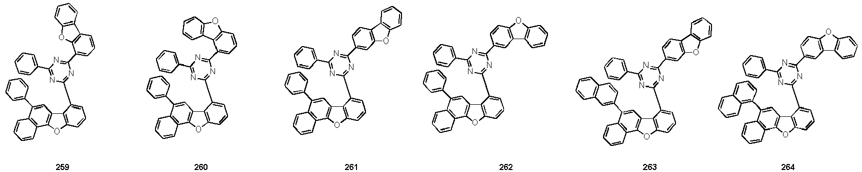

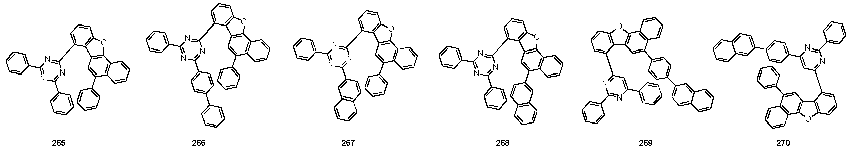

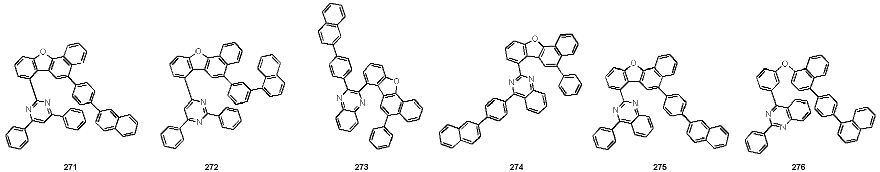

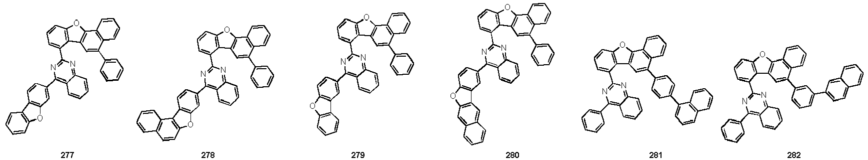

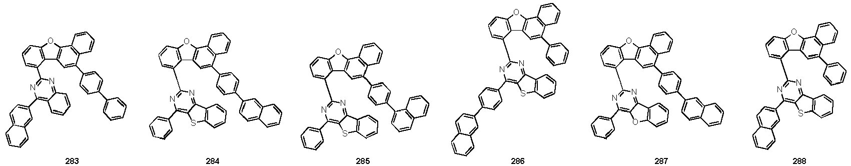

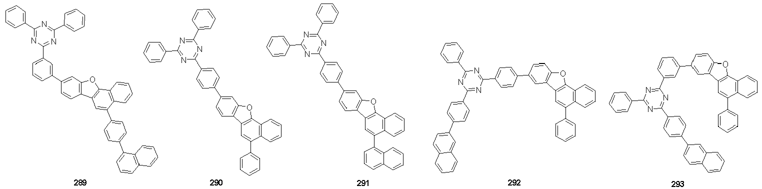

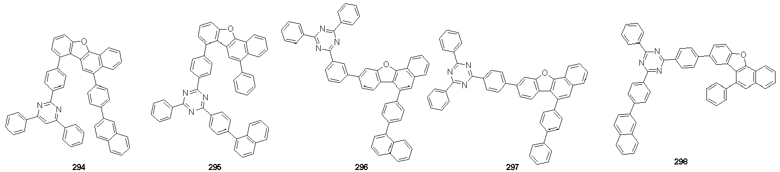

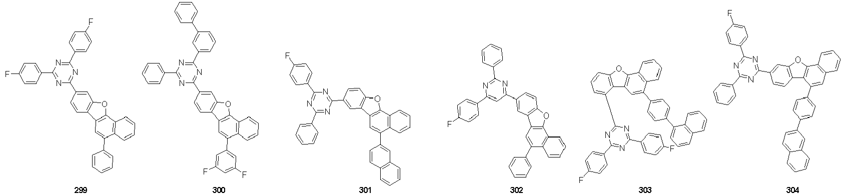

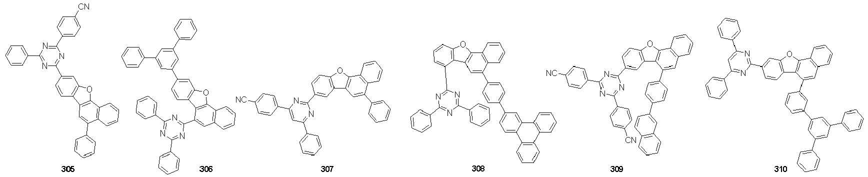

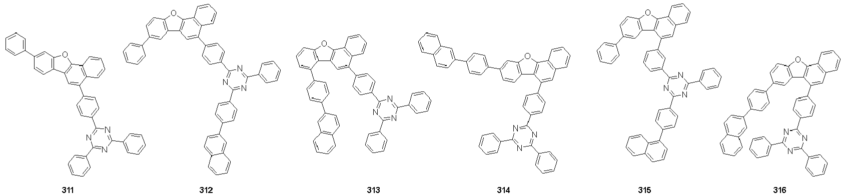

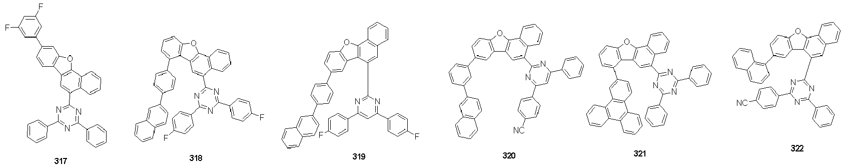

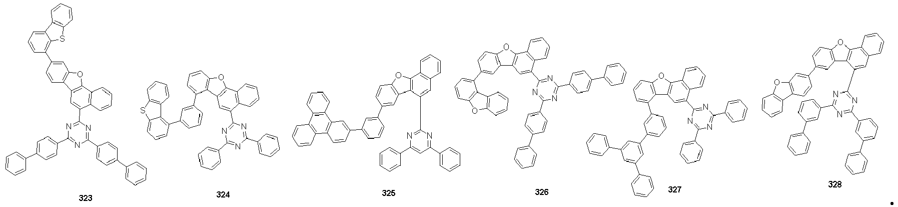

7. An organic light emitting device comprising:
a first electrode;
a second electrode; and
one or more organic material layers provided between the first electrode and the second electrode,
wherein one or more layers of the organic material layers include the heterocyclic compound of any one of Claims 1 to 6.

8. The organic light emitting device of Claim 7, wherein the organic material layer includes one or more light emitting layers, and the light emitting layer includes the heterocyclic compound.

9. The organic light emitting device of Claim 8, wherein the light emitting layer includes two or more host materials, and at least one of the host materials includes the heterocyclic compound as a host material of a light emitting material.

10. The organic light emitting device of Claim 7, further comprising one or more layers selected from the group consisting of a light emitting layer, a hole injection layer, a hole transfer layer, an electron injection layer, an electron transfer layer, a hole auxiliary layer and a hole blocking layer.

11. The organic light emitting device of Claim 7, comprising the heterocyclic compound according to Chemical Formula 1 as a first compound, and further comprising one of compounds of the following Group A to Group C as a second compound:
